# EUROPEAN PATENT APPLICATION

(11) **EP 4 800 121 A1**
(43) Date of publication of application: **02.09.2026**
(21) Application number: 24882891.5
(22) Date of filing: 25.10.2024
(51) Int. Cl.: C12Q 1/6806, C12Q 1/6869, C40B 50/06, C40B 40/08

(54) **METHOD FOR DESIGNING WIDE RANGE OF RNA MODIFICATION LIBRARY INCLUDING ALL SURROUNDING SEQUENCES OF RNA MODIFICATION AND USE THEREOF**

(30) Priority: 25.10.2023 KR 20230143795; 17.05.2024 KR 20240064626
(71) Applicant: Seoul National University R&DB Foundation, Seoul 08826 (KR)
(72) Inventor: BAEK, Daehyun, Seoul 08826 (KR); KANG, Gihyeon, Seoul 08826 (KR); CHANG, Hee Ryung, Seoul 08826 (KR); CHOI, Heejin, Seoul 08826 (KR); HWANG, Hyeonseo, Seoul 08826 (KR)
(74) Representative: Turner, Craig Robert
(86) International application number: PCT/KR2024/016386
(87) International publication number: WO 2025/089864

(57) **Abstract**

The present invention relates to a method for designing a wide range of RNA modification libraries including all sequences of RNA modification and a use thereof. The present invention can construct a modified RNA library by introducing various types of RNA modifications and can reflect all possible combinations of surrounding sequences on both sides of the modified RNA library, thereby differing from the previously reported modified RNA library construction method. In addition, when single-molecule RNA sequencing is performed using a modified RNA library according to the present invention, a high-quality reference data set including all combinatorially possible motifs can be generated. Using this dataset as training data for deep learning (or machine learning) makes it possible not only to develop new RNA modification detection software with higher accuracy, but also to simultaneously detect multiple RNA modifications in a single analysis, beyond the limitations of existing software, which are often restricted within specific RNA modifications or exhibit reduced prediction accuracy for other modifications.

## Description

### TECHNICAL FIELD

The present invention relates to a method for designing a wide range of RNA modification libraries including all flanking sequences of RNA modification and use thereof.

### BACKGROUND ART

RNA modification is a form in which a specific base of RNA is chemically changed. There are about 170 types of very diverse RNA modifications in all biological RNAs, including m⁶A (N6-methyladenosine) and m⁵C (5-methylcytidine). RNA modifications are distributed at various locations in several RNAs and have various biological roles. For example, they change base pairing, secondary structure and binding affinity with RNA binding proteins, and they also play a key role in regulating gene expression by being involved in RNA splicing and stability regulation. It has been reported that when the regulation of RNA modification is abnormal, various types of diseases, including cancer, can occur (RNA 23.12 (2017): 1754-1769 and Nature Cell Biology 21.5 (2019): 552-559). In addition, RNA modifications have been reported as regulators that play an important role in biological processes such as fertilization ability, development and cell fate determination (Cell Research 27 (2017): 1100-1114) and Cell Stem Cell 15.6 (2014): 707-719).

As the biological roles and importance of RNA modifications have been reported, attempts to detect specific RNA modifications have been increasing. However, due to the experimental and practical limitations of existing next-generation sequencing methods using antibodies, no method has been reported to accurately and broadly detect various RNA modifications at once.

Recently, synthetic RNA sequences or RNA sequences extracted from cells are used as RNA modification data for training in various models that aim to detect RNA modifications.

The synthesized RNA sequence refers to RNA created by *in-vitro* transcription (IVT). In order to create RNA including RNA modification using IVT, a specific type of RNA-modified-NTP (e.g., m⁶ATP, m⁵CTP) is used instead of NTP (ATP, CTP, GTP, UTP). For example, when m⁶ATP is used, since A on DNA is all transcribed as m⁶A instead of A in RNA, the RNA synthesized by IVT will be different from the actual biological sequence. In other words, an RNA sequence in which m⁶A exists at all A positions is created, unlike the sequence that actually exists in nature. In fact, in the case of a model in the literature Nucleic Acids Research 49.2 (2021): e7 that used such sequences as training data, it was confirmed through the literature Nature Communications 14 (2023): 1906 that the accuracy of finding RNA modifications actually existing in cells is very low. To overcome this disadvantage, in the literature Nature Communications 10.1 (2019): 4079 and Nature Biotechnology 39 (2021): 1278-1291, 5-mer sequences including A other than the middle m⁶A were excluded from the training data, and the model was trained by using only sequences that did not include other A. However, this still has the disadvantage of not considering all RNA sequences in which canonical A exists around natural m⁶A, and it was confirmed that the accuracy was still low. When it was trained by using data created using synthetic RNA that is different from naturally existing RNA or does not consider all naturally existing sequences, the model has the limitation that it cannot successfully predict m⁶A positions with various flanking sequences.

Meanwhile, when RNA extracted from cells is applied to training data, information of m⁶A present in a specific motif is used. For example, m⁶A is known to mainly exist in the middle A position of the DRACH (D = A/G/U, R = A/G, H = A/C/U) motif. In the case of the model in the literature RNA 26.1 (2020): 19-28 or Nature Methods 19.12 (2022): 1590-1598, which selected only these DR(m⁶A)CH sequences and performed training by using the same as training data, only the m⁶A present in the corresponding motif can be predicted to a limited extent, which has the limitation of not being able to widely find m⁶A actually existing in nature. In addition, for RNA modifications other than m⁶A, since the motifs that mainly exist, such as DRACH, are hardly known, the strategy of using RNA extracted from cells or using RNA modification data on a specific motif based on information derived from them is not applicable to other RNA modifications.

In this way, when using RNA modification sequences synthesized through IVT as suggested in existing research articles and models, or when using RNA modification sequences extracted from cells or sequences restricted to specific motifs, it is impossible to consider all RNA modifications present in the entire organism, and when utilizing the same as data for model training, there may be limitations in predicting the modification positions of the entire RNA. In view of this, the inventors of the present invention have made great efforts to design an RNA modification library in which all RNA modifications present in the entire organism can be reflected, and as a result, the inventors of the present invention have developed a design method for synthesizing RNA oligomers including various types of RNA modifications and all random sequences flanking the same at a desired position by a chemical or template-independent enzymatic synthesis method and using the same to produce an RNA modification library, thereby completing the present invention.

### DISCLOSURE

### TECHNICAL PROBLEM

The present invention has an object to provide a method for producing an RNA modification library based on a broad library design method of RNA modifications including combinations of all flanking sequences of RNA modifications and use thereof.

### TECHNICAL SOLUTION

In order to achieve the above object, the present invention provides a method for producing an RNA modification library, including the following steps:
(a) preparing one or more modified RNA blocks in which 4 to 50 random nucleotides are randomly linked to each of 5' and 3' directions of a modified RNA;
(b) preparing a modified RNA oligomer in which 1 to 9 modified RNA blocks are randomly arranged among the one or more modified RNA blocks;
(c) ligating the modified RNA oligomers to prepare a modified RNA oligomer concatemer;
(d) selecting a modified RNA oligomer concatemer in which 2 to 20 of the modified RNA oligomers are ligated;
(e) attaching an RNA tail consisting of 5 to 150 arbitrary ribonucleotides to the selected modified RNA oligomer concatemer; and
(f) recovering a modified RNA oligomer concatemer to which the RNA tail is attached.

In the present invention, the modified RNA may be
any one modified RNA selected from the group consisting of:
(a) A modification of Table [1] below:

**[Table 1]**

| | **Name** | Short **Name** |
|---|---|---|
| 1 | 2-methylthio-N6-methyladenosine | ms²m⁶A |
| 2 | N6-(cis-hydroxyisopentenyl)adenosine | io⁶A |
| 3 | N6-methyl-N6-threonylcarbamoyladenosine | m6t⁶A |
| 4 | N6-hydroxynorvalylcarbamoyladenosine | hn⁶A |
| 5 | 2'-O-ribosyladenosine (phosphate) | Ar(p) |
| 6 | 1-methylinosine | m¹I |
| 7 | 1,2'-O-dimethyladenosine | m¹Am |
| 8 | 1,2'-O-dimethylinosine | m¹Im |
| 9 | 2-methyladenosine | m²A |
| 10 | N6,N6-dimethyladenosine | m⁶₂A |
| 11 | 2-methylthio-N6-isopentenyladenosine | ms²i⁶A |
| 12 | 2-methylthio-N6-threonylcarbamoyladenosine | ms²t⁶A |
| 13 | N6-isopentenyladenosine | i⁶A |
| 14 | 2-methylthio-N6-hydroxynorvalylcarbamoyladenosine | ms²hn⁶A |
| 15 | N6,2'-O-dimethyladenosine | m⁶Am |
| 16 | 1-methyladenosine | m¹A |
| 17 | N6-methyladenosine | m⁶A |
| 18 | inosine | I |
| 19 | N6-glycinylcarbamoyladenosine | g⁶A |
| 20 | N6,N6,2'-O-trimethyladenosine | m⁶₂Am |
| 21 | N6-acetyladenosine | ac⁶A |
| 22 | 2-methylthio-N6-(cis-hydroxyisopentenyl) adenosine | ms²io⁶A |
| 23 | 2'-O-methyladenosine | Am |
| 24 | 2'-O-methylinosine | Im |
| 25 | N6-threonylcarbamoyladenosine | t⁶A |
| 26 | 8-methyladenosine | m³A |
| 27 | 2,8-dimethyladenosine | m^{2,8}A |
| 28 | cyclic N6-threonylcarbamoyladenosine | ct⁶A |
| 29 | N6-hydroxymethyladenosine | hm⁶A |
| 30 | N6-formyladenosine | f⁶A |
| 31 | 2-methylthio cyclic N6-threonylcarbamoyladenosine | ms²ct⁶A |
| 32 | hydroxy-N6-threonylcarbamoyladenosine | ht⁶A |
| 33 | 2- methylthiomethylenethio-N6-isopentenyl-adenosine | msms²i⁶A |

(2) C modification of [Table 2] below:

**[Table 2]**

| | **Name** | **Short Name** |
|---|---|---|
| 1 | 5,2'-O-dimethylcytidine | m⁵Cm |
| 2 | N4-methylcytidine | m⁴C |
| 3 | N4,N4-dimethylcytidine | m⁴₂C |
| 4 | 5-methylcytidine | m⁵C |
| 5 | 5-formylcytidine | f⁵C |
| 6 | N4,2'-O-dimethylcytidine | m⁴Cm |
| 7 | 3-methylcytidine | m³C |
| 8 | N4,N4,2'-O-trimethylcytidine | m⁴₂Cm |
| 9 | N4-acetylcytidine | ac⁴C |
| 10 | 2'-O-methylcytidine | Cm |
| 11 | 2-thiocytidine | s²C |
| 12 | 2-lysidine | k²C |
| 13 | 5-formyl-2'-O-methylcytidine | f⁵C^{m} |
| 14 | N4-acetyl-2'-O-methylcytidine | ac⁴Cm |
| 15 | 5-hydroxymethylcytidine | hm⁵C |
| 16 | agmatidine | C+ |
| 17 | 5-hydroxycytidine | ho⁵C |
| 18 | 2'-O-methyl-5-hydroxymethylcytidine | hm⁵Cm |

(3) G modification of [Table 3] below:

**[Table 3]**

| | **Name** | **Short Name** |
|---|---|---|
| 1 | N2,7-dimethylguanosine | m^{2,7}G |
| 2 | epoxyqueuosine | oQ |
| 3 | N2,N2-dimethylguanosine | m²₂G |
| 4 | 1-methylguanosine | m¹G |
| 5 | 2'-O-methylguanosine | Gm |
| 6 | wybutosine | yW |
| 7 | 2'-O-ribosylguanosine (phosphate) | Gr(p) |
| 8 | N2-methylguanosine | m²G |
| 9 | N2,N2,2'-O-trimethylguanosine | m²₂Gm |
| 10 | glutamyl-queuosine | gluQ |
| 11 | methylwyosine | mimG |
| 12 | 7-aminomethyl-7-carbaguanine | preQ1base |
| 13 | 1,2'-O-dimethylguanosine | m¹Gm |
| 14 | queuosine | Q |
| 15 | 7-aminocarboxypropyl-demethylwyosine | yW-86 |
| 16 | 7-aminocarboxypropylwyosine | yW-72 |
| 17 | galactosyl-queuosine | galQ |
| 18 | isowyosine | imG2 |
| 19 | mannosyl-queuosine | manQ |
| 20 | hydroxywybutosine | OHyW |
| 21 | archaeosine | G+ |
| 22 | N2,2'-O-dimethylguanosine | m²Gm |
| 23 | 4-demethylwyosine | imG-14 |
| 24 | N2,N2,7-trimethylguanosine | m²₂⁷G |
| 25 | 7-aminocarboxypropylwyosine methyl ester | yW-58 |
| 26 | 7-methylguanosine | m⁷G |
| 27 | 7-cyano-7-deazaguanosine | preQ0 |
| 28 | peroxywybutosine | o₂yW |
| 29 | 7-aminomethyl-7-deazaguanosine | preQ1 |
| 30 | wyosine | imG |
| 31 | N2,7,2'-O-trimethylguanosine | m^{2,7}Gm |

and
(4) U modification of [Table 4] below:

**[Table 4]**

| | **Name** | **Short Name** |
|---|---|---|
| 1 | 5-formyluridine | f⁵U |
| 2 | 5-methyl-2-thiouridine | m⁵s²U |
| 3 | 4-thiouridine | s⁴U |
| 4 | 1-methyl-3-(3-amino-3-carboxypropyl)pseudouridine | m¹acp³Y |
| 5 | 5-carboxymethyl-2-thiouridine | cm⁵s²U |
| 6 | 5-methyluridine | m⁵U |
| 7 | dihydrouridine | D |
| 8 | 5-methyldihydrouridine | m⁵D |
| 9 | 5-taurinomethyluridine | tm⁵U |
| 10 | 5-carboxymethylaminomethyl-2'-O-methyluridine | cmnm⁵Um |
| 11 | 5-formyl-2' | f⁵Um |
| 12 | 5-methoxycarbonylmethyluridine | mcm⁵U |
| 13 | 5-carbamoylmethyl-2'-O-methyluridine | ncm⁵Um |
| 14 | 5-aminomethyl-2-thiouridine | nm⁵s²U |
| 15 | 5-carbamoylmethyluridine | ncm⁵U |
| 16 | 5-carboxymethyluridine | cm⁵U |
| 17 | 5-carboxymethylaminomethyl-2-thiouridine | cmnm⁵s²U |
| 18 | uridine 5-oxyacetic acid | cmo⁵U |
| 19 | 3-methylpseudouridine | m³Y |
| 20 | 3-methyluridine | m³U |
| 21 | 2'-O-methyluridine | Um |
| 22 | 3-(3-amino-3-carboxypropyl)methyluridine | acp³Um |
| 23 | 5-carboxymethylaminomethyl-2-selenouridine | cmnm⁵se²U |
| 24 | 3,2'-O-dimethyluridine | m³Um |
| 25 | uridine 5-oxyacetic acid methyl ester | mcmo⁵U |
| 26 | 5-aminomethyl-2-selenouridine | nm⁵se²U |
| 27 | 5-hydroxyuridine | ho⁵U |
| 28 | 5-formyl-2-thiouridine | f⁵s²U |
| 29 | 5-methoxycarbonylmethyl-2-thiouridine | mcm⁵s²U |
| 30 | 5-carboxyhydroxymethyluridine | chm⁵U |
| 31 | 5-methoxyuridine | mo⁵U |
| 32 | 3-(3-amino-3-carboxypropyl)uridine | acp³U |
| 33 | 5-methylaminomethyl-2-selenouridine | mnm⁵se²U |
| 34 | 5-methoxycarbonylmethyl-2'-O-methyluridine | mcm⁵Um |
| 35 | 5-(carboxyhydroxymethyl)uridine methyl ester | mchm⁵U |
| 36 | 5-carboxymethylaminomethyluridine | cmnm⁵U |
| 37 | 1-methylpseudouridine | m¹Y |
| 38 | 5-taurinomethyl-2-thiouridine | tm⁵s²U |
| 39 | 2-selenouridine | se²U |
| 40 | 2-thiouridine | s²U |
| 41 | 2'-O-methylpseudouridine | Ym |
| 42 | 5-formyl-2-selenouridine | f⁵se²U |
| 43 | 5-methylaminomethyl-2-thiouridine | mnm⁵s²U |
| 44 | 5-aminomethyluridine | nm⁵U |
| 45 | 2-thio-2'-O-methyluridine | s²Um |
| 46 | pseudouridine | Y |
| 47 | 5,2'-O-dimethyluridine | m⁵Um |
| 48 | 5-methylaminomethyluridine | mnm⁵U |
| 49 | 3-(3-amino-3-carboxypropyl)-5,6-dihydrouridine | acp³D |
| 50 | 3-(3-amino-3-carboxypropyl)pseudouridine | acp³Y |
| 51 | 5-(isopentenylaminomethyl)uridine | inm⁵U |
| 52 | 5-(isopentenylaminomethyl)-2'-O-methyluridine | inm⁵Um |
| 53 | 5-(isopentenylaminomethyl)-2-thiouridine | inm⁵s²U |
| 54 | 5-carbamoylmethyl-2-thiouridine | ncm⁵s²U |
| 55 | 5-carbamoylhydroxymethyluridine | nchm⁵U |
| 56 | 5-(carboxyhydroxymethyl)-2'-O-methyluridine methyl ester | mchm⁵Um |
| 57 | 2-geranylthiouridine | ges²U |
| 58 | 5-carboxymethylaminomethyl-2-geranylthiouridine | cmnm⁵ges²U |
| 59 | 5-methylaminomethyl-2-geranylthiouridine | mnm⁵ges²U |
| 60 | 5-aminomethyl-2-geranylthiouridine | nm⁵ges²U |
| 61 | 5-cyanomethyluridine | cnm⁵U |
| 62 | 2'-O-methyluridine 5-oxyacetic acid methyl ester | mcmo⁵Um |

In the present invention, step (b) may be producing a modified RNA oligomer, further including an anchor sequence
(i) between each modified RNA block constituting a modified RNA oligomer,
(ii) at a 5' end of a modified RNA block located at a 5' end of a modified RNA block constituting a modified RNA oligomer, and/or
(iii) at a 3' end of a modified RNA block located at a 3' end of a modified RNA block constituting a modified RNA oligomer.

In the present invention, the anchor sequence may have a sequence of 4 nucleotides or more, and when there are 2 or less modified RNA blocks in the modified RNA oligomer, the anchor sequence has a length of 40 to 90% of a length of a modified RNA block, and when there are 3 to 9 modified RNA blocks in the modified RNA oligomer, the anchor sequence has a length of 20 to 30% of a length of a modified RNA block.

In the present invention, step (c) may be ligating with an RNA ligase.

In the present invention, step (b) may be producing by additionally introducing 2 to 4 specified nucleotide combinations to a 5' end and 3' end of the modified RNA oligomer, respectively.

In the present invention, the RNA ligase may be T4 RNA ligase 1, the 2 specified nucleotide combinations may be any one of combinations listed in Table 5 below, the 3 specified nucleotide combinations may be any one of combinations listed in Table 6 below, and the 4 specified nucleotide combinations may be any one of the combinations listed in Table 7 below,

**[Table 5]**

| No | 5' end | 3' end |
|---|---|---|
| 1 | GG | GG |
| 2 | GA | AC |
| 3 | GA | CC |
| 4 | CG | GA |
| 5 | CG | UC |
| 6 | UC | AG |
| 7 | AC | CG |
| 8 | GU | CA |
| 9 | AU | CG |
| 10 | UC | GA |

**[Table 6]**

| No | 5' end | 3' end |
|---|---|---|
| 1 | GAU | GAC |
| 2 | CGA | CGA |
| 3 | CGA | GUC |
| 4 | AUC | ACG |
| 5 | GAC | UCC |
| 6 | CCG | AGU |
| 7 | ACG | CCG |
| 8 | UCC | CAG |
| 9 | GUC | ACA |
| 10 | AGU | UAC |

**[Table 7]**

| No | 5' end | 3' end |
|---|---|---|
| 1 | CGAC | AGUC |
| 2 | GAUC | CGAC |
| 3 | GACG | GUCC |
| 4 | CGAU | CCGA |
| 5 | ACGA | UCCG |
| 6 | CCGA | CAGU |
| 7 | UCCG | ACAG |
| 8 | GUCC | UACA |
| 9 | AGUC | CUAC |
| 10 | CAGU | UCUA |

wherein the RNA ligase may be T4 RNA ligase 2 truncated KQ, the 2 specified nucleotide combinations may be any one of combinations listed in Table 8 below, the 3 specified nucleotide combinations may be any one of combinations listed in Table 9 below, and the 4 specified nucleotide combinations may be any one of combinations listed in Table 10 below:

**[Table 8]**

| No | 5' end | 3' end |
|---|---|---|
| 1 | AG | CG |
| 2 | GG | UU |
| 3 | GA | CC |
| 4 | GA | UG |
| 5 | AA | GC |
| 6 | GG | CU |
| 7 | GA | CU |
| 8 | GA | CG |
| 9 | GC | UG |
| 10 | GA | GC |

**[Table 9]**

| No | 5' end | 3' end |
|---|---|---|
| 1 | AGA | CCG |
| 2 | AGA | UCG |
| 3 | GCA | UUG |
| 4 | GGC | CUU |
| 5 | GGA | CUU |
| 6 | AGA | ACG |
| 7 | GAG | CCC |
| 8 | GAA | CCC |
| 9 | GCA | CUG |
| 10 | AGA | GCG |

**[Table 10]**

| No | 5' end | 3' end |
|---|---|---|
| 1 | AGAA | CUCG |
| 2 | GGCA | CCUU |
| 3 | AGAA | ACCG |
| 4 | AGAA | UCCG |
| 5 | GGCA | ACUU |
| 6 | AGAA | UUCG |
| 7 | AGAA | GCCG |
| 8 | GGCA | UCUU |
| 9 | AGAA | CACG |
| 10 | AGAA | AUCG |

In the present invention, steps (a) and (b) may be produced by chemical or template-independent enzymatic synthesis, and step (b) may be produced as a separate process after step (a), or steps (a) and (b) may be produced as a single process.

In the present invention, step (c) may be ligating at 29°C to 37°C for 8 to 40 hours.

In the present invention, step (c) may be ligating in a reaction solution including 13 to 21% (v/v) of PEG8000.

In the present invention, the RNA tail of step (e) may be a poly(A) tail, poly(U) tail or poly(I) tail consisting of 5 to 40 ribonucleotides.

In the present invention, the poly(A) tail, poly(U) tail or poly(I) tail of step (e) may be reacted in a reaction solution including 5 to 50 uM of ATP, UTP or ITP, respectively.

In addition, the present invention provides an RNA modification library, including a plurality of modified RNA oligomer concatemers in the form of 2 to 20 ligated modified RNA oligomers,
wherein the modified RNA oligomer is characterized in that 1 to 9 modified RNA blocks in which 4 to 50 random nucleotides are randomly linked in 5' and 3' directions, respectively, are randomly arranged consecutively around modified RNA as a center.

In the present invention, the modified RNA may be
any one of modified RNA selected from the group consisting of:
(1) A modification of [Table 1] above:
(2) C modification of [Table 2] above:
(3) G modification of [Table 3] above: and
(4) U modification of [Table 4] below.

In the present invention, the modified RNA oligomer may further include an anchor sequence
(i) between each modified RNA block constituting a modified RNA oligomer,
(ii) at a 5' end of a modified RNA block located at a 5' end of a modified RNA block constituting a modified RNA oligomer, and/or
(iii) at a 3' end of a modified RNA block located at a 3' end of a modified RNA block constituting a modified RNA oligomer.

In the present invention, the anchor sequence may have a sequence of 4 nucleotides or more, and when there are 2 or less modified RNA blocks in the modified RNA oligomer, the anchor sequence has a length of 40 to 90% of a length of a modified RNA block, and when there are 3 to 9 modified RNA blocks in the modified RNA oligomer, the anchor sequence has a length of 20 to 30% of a length of a modified RNA block.

In the present invention, 2 to 4 specified nucleotide combinations may be introduced to a 5' end and 3' end of the modified RNA oligomer, respectively.

In the present invention, the modified RNA oligomer concatemer may have an additional RNA tail consisting of 5 to 150 arbitrary ribonucleotides attached to a 3' end.

In addition, the present invention provides an RNA library set for training an RNA modification detection model, including: the RNA modification library; and
a canonical RNA library including a plurality of canonical RNA oligomer concatemers having the same structure as a plurality of modified RNA oligomer concatemers included in the RNA modification library, except that the modified RNA is replaced with a corresponding canonical RNA.

In addition, the present invention provides a sequencing data set, generated by using the RNA library set for training an RNA modification detection model.

In the present invention, the sequencing may be single-molecule RNA sequencing.

In the present invention, the sequencing data set may be for deep learning and/or machine learning training.

### ADVANTAGEOUS EFFECTS

The present invention is distinguished from the previously reported method for producing an RNA modification library in that it is possible to produce an RNA modification library by introducing various types of RNA modifications, and can reflect all possible combinations of flanking sequences on both sides of a centered modified RNA. In addition, when single-molecule RNA sequencing is performed by using the RNA modification library according to the present invention, a high-quality reference data set including all combinatorially possible motifs can be generated, and when this is utilized as training data for deep learning (or machine learning), it will be possible to develop new RNA modification detection software with higher accuracy, overcoming the limitations of previously invented software that are limited to specific RNA modifications or have low prediction accuracy for other modifications, and it will also be possible to detect multiple RNA modifications simultaneously.

### DESCRIPTION OF DRAWINGS

FIG. 1 is an exemplary illustration of a modified RNA oligomer produced according to some embodiments of the present invention and a corresponding canonical RNA oligomer.
FIG. 2 schematically illustrates an experiment for producing an RNA modification library according to the present invention and a corresponding canonical RNA library.
FIG. 3a shows the ranking of ligation efficiency according to the sequence combination of both ends of an RNA oligomer in the step of ligating the RNA oligomer according to the present invention with T4 RNA ligase1.
FIG. 3b shows the ranking of ligation efficiency according to the sequence combination of both ends of an RNA oligomer in the step of ligating the RNA oligomer according to the present invention with T4 RNA ligase 2 truncated KQ.
FIG. 4a schematically illustrates an experiment in which an RNA oligomer according to the present invention is ligated with various RNA ligases; RNA ligase 1, RNA ligase 2 truncated KQ, and RNA ligase 2.
FIG. 4b is a PAGE result after ligation of an RNA oligomer according to the present invention with various RNA ligases; RNA ligase 1, RNA ligase 2 truncated KQ, and RNA ligase 2.
FIG. 5a shows the results of determining the ligation efficiency according to a reaction temperature in the step of ligating an RNA oligomer according to the present invention with T4 RNA ligase1.
FIG. 5b shows the results of determining the ligation efficiency according to the concentration of PEG8000 in the step of ligating an RNA oligomer according to the present invention with T4 RNA ligase1.
FIG. 5c shows the results of determining the ligation efficiency according to the concentration of DMSO in the step of ligating an RNA oligomer according to the present invention with T4 RNA ligase1.
FIG. 5d shows the results of determining the ligation efficiency according to the reaction time in the step of ligating the RNA oligomer according to the present invention with T4 RNA ligase1.
FIG. 5e shows the results of comparing the RNA oligomer ligation efficiency of the optimized conditions established for ligating the RNA oligomer according to the present invention and the conventional conditions for ligation using T4 RNA ligase 1.
FIG. 6 shows the results of determining the optimized length of poly(A) tailing in nanopore sequencing by using an RNA modification library produced according to the present invention.
FIG. 7a shows the results of determining the ATP concentration for poly(A) tailing of an optimized length in an RNA modification library produced according to the present invention.
FIG. 7b shows the results of confirming the amount of E-PAP added and the reaction time for poly(A) tailing of an optimized length in an RNA modification library produced according to the present invention.
FIG. 7c is a result confirming that attachment of an RNA tail with an accurate length through ligation is possible in the production of an RNA modification library of the present invention.
FIG. 7d shows the result of producing an RNA modification library by attaching an RNA tail through ligation of App-RNA to a modified RNA oligomer concatemer produced according to the present invention.
FIG. 8a shows an actual example of a single-molecule sequencing performed by using an m⁶A library and a canonical A library produced according to the present invention, and the results (number of reads, number of bases).
FIG. 8b shows an actual example of a single-molecule sequencing performed by using an m⁵C library and a canonical C library produced according to the present invention, and the results (number of reads, number of bases).
FIG. 9a shows the results of determining base quality according to the position of the modified RNA in motifs extracted from sequencing data of the m⁶A library produced according to the present invention and the canonical A library.
FIG. 9b shows the results of determining the average value of current signals according to the position of the modified RNA in the motif extracted from the sequencing data of the m⁶A library produced according to the present invention and the canonical A library.
FIG. 9c shows the results of determining the difference in current signals according to motif in the motif extracted from the sequencing data of the m⁶A library produced according to the present invention and the canonical A library.
FIG. 10a shows the results comparing the degree of improvement in RNA oligomer design according to the anchor insertion method.
FIG. 10b shows the results comparing the motif extraction accuracy according to the number of RNA blocks and the length of the anchor sequence.
FIG. 10c is an example of inserting a derived anchor sequence between blocks in the design of the modified RNA oligomer and the corresponding canonical RNA oligomer.

### MODES OF THE INVENTION

Unless otherwise defined, all technical and scientific terms used in the present specification have the same meanings as commonly understood by one of ordinary skill in the art to which this invention pertains. In general, the nomenclature used in the present specification and the experimental methods described below are well known and commonly used in the art.

In the present invention, it should be understood that terms such as "include" or "have" are intended to specify the presence of a feature, number, step, operation, component, part or combination thereof described in the specification, but do not exclude in advance the possibility of the presence or addition of one or more other features, numbers, steps, operations, components, parts or combinations thereof.

In the present invention, a method for designing a wide range of RNA modification library including all combinations of flanking sequences of RNA modification has been developed. Specifically, in the present invention, RNA oligomers including various types of RNA modifications at desired positions and all random sequences flanking the same were designed and chemically synthesized, and an RNA modification library was produced by using the same.

Accordingly, the present invention relates, in one aspect, to a method for producing an RNA modification library including the following steps:
(a) preparing one or more modified RNA blocks in which 4 to 50 random nucleotides are randomly linked to each of 5' and 3' directions of a modified RNA;
(b) preparing a modified RNA oligomer in which 1 to 9 modified RNA blocks are randomly arranged among the one or more modified RNA blocks;
(c) ligating the modified RNA oligomers to prepare a modified RNA oligomer concatemer;
(d) selecting a modified RNA oligomer concatemer in which 2 to 20 of the modified RNA oligomers are ligated;
(e) attaching an RNA tail consisting of 5 to 150 arbitrary ribonucleotides to the selected modified RNA oligomer concatemer; and
(f) recovering a modified RNA oligomer concatemer to which the RNA tail is attached.

In one aspect, the method for producing an RNA modification library may include the following steps:
(a) preparing one or more modified RNA blocks in which 4 to 50 random nucleotides are randomly linked to each of 5' and 3' directions of a modified RNA;
(b) preparing a modified RNA oligomer in which 1 to 9 modified RNA blocks are randomly arranged among the one or more modified RNA blocks;
(c) ligating the modified RNA oligomers to prepare a modified RNA oligomer concatemer;
(d) selecting a modified RNA oligomer concatemer in which 2 to 20 of the modified RNA oligomers are ligated;
(e) attaching an RNA tail consisting of 5 to 150 arbitrary ribonucleotides to the selected modified RNA oligomer concatemer; and
(f) recovering a modified RNA oligomer concatemer to which the RNA tail is attached.

In the present invention, the modified RNA may be
any one modified RNA selected from the group consisting of:
(a) A modification of Table [1] below:

**[Table 1]**

| | **Name** | **Short Name** |
|---|---|---|
| 1 | 2-methylthio-N6-methyladenosine | ms²m⁶A |
| 2 | N6-(cis-hydroxyisopentenyl)adenosine | io⁶A |
| 3 | N6-methyl-N6-threonylcarbamoyladenosine | m6t⁶A |
| 4 | N6-hydroxynorvalylcarbamoyladenosine | hn⁶A |
| 5 | 2'-O-ribosyladenosine (phosphate) | Ar(p) |
| 6 | 1-methylinosine | m¹I |
| 7 | 1,2'-O-dimethyladenosine | m¹Am |
| 8 | 1,2'-O-dimethylinosine | m¹Im |
| 9 | 2-methyladenosine | m²A |
| 10 | N6,N6-dimethyladenosine | m⁶₂A |
| 11 | 2-methylthio-N6-isopentenyladenosine | ms²i⁶A |
| 12 | 2-methylthio-N6-threonylcarbamoyladenosine | ms²t⁶A |
| 13 | N6-isopentenyladenosine | i⁶A |
| 14 | 2-methylthio-N6-hydroxynorvalylcarbamoyladenosine | ms²hn⁶A |
| 15 | N6,2'-O-dimethyladenosine | m⁶Am |
| 16 | 1-methyladenosine | m¹A |
| 17 | N6-methyladenosine | m⁶A |
| 18 | inosine | I |
| 19 | N6-glycinylcarbamoyladenosine | g⁶A |
| 20 | N6,N6,2'-O-trimethyladenosine | m⁶₂Am |
| 21 | N6-acetyladenosine | ac⁶A |
| 22 | 2-methylthio-N6-(cis-hydroxyisopentenyl) adenosine | ms²io⁶A |
| 23 | 2'-O-methyladenosine | Am |
| 24 | 2'-O-methylinosine | Im |
| 25 | N6-threonylcarbamoyladenosine | t⁶A |
| 26 | 8-methyladenosine | m³A |
| 27 | 2,8-dimethyladenosine | m^{2,8}A |
| 28 | cyclic N6-threonylcarbamoyladenosine | ct⁶A |
| 29 | N6-hydroxymethyladenosine | hm⁶A |
| 30 | N6-formyladenosine | f⁶A |
| 31 | 2-methylthio cyclic N6-threonylcarbamoyladenosine | ms²ct⁶A |
| 32 | hydroxy-N6-threonylcarbamoyladenosine | ht⁶A |
| 33 | 2- methylthiomethylenethio-N6-isopentenyl-adenosine | msms²i⁶A |

(2) C modification of [Table 2] below:

**[Table 2]**

| | **Name** | **Short Name** |
|---|---|---|
| 1 | 5,2'-O-dimethylcytidine | m⁵Cm |
| 2 | N4-methylcytidine | m⁴C |
| 3 | N4,N4-dimethylcytidine | m⁴₂C |
| 4 | 5-methylcytidine | m⁵C |
| 5 | 5-formylcytidine | f⁵C |
| 6 | N4,2'-O-dimethylcytidine | m⁴Cm |
| 7 | 3-methylcytidine | m³C |
| 8 | N4,N4,2'-O-trimethylcytidine | m⁴₂Cm |
| 9 | N4-acetylcytidine | ac⁴C |
| 10 | 2'-O-methylcytidine | Cm |
| 11 | 2-thiocytidine | s²C |
| 12 | 2-lysidine | k²C |
| 13 | 5-formyl-2'-O-methylcytidine | f⁵C^{m} |
| 14 | N4-acetyl-2'-O-methylcytidine | ac⁴Cm |
| 15 | 5-hydroxymethylcytidine | hm⁵C |
| 16 | agmatidine | C+ |
| 17 | 5-hydroxycytidine | ho⁵C |
| 18 | 2'-O-methyl-5-hydroxymethylcytidine | hm⁵Cm |

(3) G modification of [Table 3] below:

**[Table 3]**

| | **Name** | **Short Name** |
|---|---|---|
| 1 | N2,7-dimethylguanosine | m^{2,7}G |
| 2 | epoxyqueuosine | oQ |
| 3 | N2,N2-dimethylguanosine | m²₂G |
| 4 | 1-methylguanosine | m¹G |
| 5 | 2'-O-methylguanosine | Gm |
| 6 | wybutosine | yW |
| 7 | 2'-O-ribosylguanosine (phosphate) | Gr(p) |
| 8 | N2-methylguanosine | m²G |
| 9 | N2,N2,2'-O-trimethylguanosine | m²₂Gm |
| 10 | glutamyl-queuosine | gluQ |
| 11 | methylwyosine | mimG |
| 12 | 7-aminomethyl-7-carbaguanine | preQ1base |
| 13 | 1,2'-O-dimethylguanosine | m¹Gm |
| 14 | queuosine | Q |
| 15 | 7-aminocarboxypropyl-demethylwyosine | yW-86 |
| 16 | 7-aminocarboxypropylwyosine | yW-72 |
| 17 | galactosyl-queuosine | galQ |
| 18 | isowyosine | imG2 |
| 19 | mannosyl-queuosine | manQ |
| 20 | hydroxywybutosine | OHyW |
| 21 | archaeosine | G+ |
| 22 | N2,2'-O-dimethylguanosine | m²Gm |
| 23 | 4-demethylwyosine | imG-14 |
| 24 | N2,N2,7-trimethylguanosine | m²₂⁷G |
| 25 | 7-aminocarboxypropylwyosine methyl ester | yW-58 |
| 26 | 7-methylguanosine | m⁷G |
| 27 | 7-cyano-7-deazaguanosine | preQ0 |
| 28 | peroxywybutosine | o₂yW |
| 29 | 7-aminomethyl-7-deazaguanosine | preQ1 |
| 30 | wyosine | imG |
| 31 | N2,7,2'-O-trimethylguanosine | m^{2,7}Gm |

and
(4) U modification of [Table 4] below:

**[Table 4]**

| | **Name** | **Short Name** |
|---|---|---|
| 1 | 5-formyluridine | f⁵U |
| 2 | 5-methyl-2-thiouridine | m⁵s²U |
| 3 | 4-thiouridine | 5⁴U |
| 4 | 1-methyl-3-(3-amino-3-carboxypropyl)pseudouridine | m¹acp³Y |
| 5 | 5-carboxymethyl-2-thiouridine | cm⁵s²U |
| 6 | 5-methyluridine | m⁵U |
| 7 | dihydrouridine | D |
| 8 | 5-methyldihydrouridine | m⁵D |
| 9 | 5-taurinomethyluridine | tm⁵U |
| 10 | 5-carboxymethylaminomethyl-2'-O-methyluridine | cmnm⁵Um |
| 11 | 5-formyl-2' | f⁵Um |
| 12 | 5-methoxycarbonylmethyluridine | mcm⁵U |
| 13 | 5-carbamoylmethyl-2'-O-methyluridine | ncm⁵Um |
| 14 | 5-aminomethyl-2-thiouridine | nm⁵s²U |
| 15 | 5-carbamoylmethyluridine | ncm⁵U |
| 16 | 5-carboxymethyluridine | cm⁵U |
| 17 | 5-carboxymethylaminomethyl-2-thiouridine | cmnm⁵s²U |
| 18 | uridine 5-oxyacetic acid | cmo⁵U |
| 19 | 3-methylpseudouridine | m³Y |
| 20 | 3-methyluridine | m³U |
| 21 | 2'-O-methyluridine | Um |
| 22 | 3-(3-amino-3-carboxypropyl)methyluridine | acp³Um |
| 23 | 5-carboxymethylaminomethyl-2-selenouridine | cmnm⁵se²U |
| 24 | 3,2'-O-dimethyluridine | m³Um |
| 25 | uridine 5-oxyacetic acid methyl ester | mcmo⁵U |
| 26 | 5-aminomethyl-2-selenouridine | nm⁵se²U |
| 27 | 5-hydroxyuridine | ho⁵U |
| 28 | 5-formyl-2-thiouridine | f⁵s²U |
| 29 | 5-methoxycarbonylmethyl-2-thiouridine | mcm⁵s²U |
| 30 | 5-carboxyhydroxymethyluridine | chm⁵U |
| 31 | 5-methoxyuridine | mo⁵U |
| 32 | 3-(3-amino-3-carboxypropyl)uridine | acp³U |
| 33 | 5-methylaminomethyl-2-selenouridine | mnm⁵se²U |
| 34 | 5-methoxycarbonylmethyl-2'-O-methyluridine | mcm⁵Um |
| 35 | 5-(carboxyhydroxymethyl)uridine methyl ester | mchm⁵U |
| 36 | 5-carboxymethylaminomethyluridine | cmnm⁵U |
| 37 | 1-methylpseudouridine | m¹Y |
| 38 | 5-taurinomethyl-2-thiouridine | tm⁵s²U |
| 39 | 2-selenouridine | se²U |
| 40 | 2-thiouridine | s²U |
| 41 | 2'-O-methylpseudouridine | Ym |
| 42 | 5-formyl-2-selenouridine | f⁵se²U |
| 43 | 5-methylaminomethyl-2-thiouridine | mnm⁵s^{Z}U |
| 44 | 5-aminomethyluridine | nm⁵U |
| 45 | 2-thio-2'-O-methyluridine | s²Um |
| 46 | pseudouridine | Y |
| 47 | 5,2'-O-dimethyluridine | m⁵Um |
| 48 | 5-methylaminomethyluridine | mnm⁵U |
| 49 | 3-(3-amino-3-carboxypropyl)-5,6-dihydrouridine | acp³D |
| 50 | 3-(3-amino-3-carboxypropyl)pseudouridine | acp³Y |
| 51 | 5-(isopentenylaminomethyl)uridine | inm⁵U |
| 52 | 5-(isopentenylaminomethyl)-2'-O-methyluridine | inm⁵Um |
| 53 | 5-(isopentenylaminomethyl)-2-thiouridine | inm⁵s²U |
| 54 | 5-carbamoylmethyl-2-thiouridine | ncm⁵s²U |
| 55 | 5-carbamoylhydroxymethyluridine | nchm⁵U |
| 56 | 5-(carboxyhydroxymethyl)-2'-O-methyluridine methyl ester | mchm⁵Um |
| 57 | 2-geranylthiouridine | ges²U |
| 58 | 5-carboxymethylaminomethyl-2-geranylthiouridine | cmnm⁵ges²U |
| 59 | 5-methylaminomethyl-2-geranylthiouridine | mnm⁵ges²U |
| 60 | 5-aminomethyl-2-geranylthiouridine | nm⁵ges²U |
| 61 | 5-cyanomethyluridine | cnm⁵U |
| 62 | 2'-O-methyluridine 5-oxyacetic acid methyl ester | mcmo⁵Um |

That is, in the present invention, the modified RNA may be any one of various modified RNAs listed in Tables 1 to 4 above.

In step (a) above, any number of nucleotides, which are the same or different, may be randomly combined in both directions of the modified RNA, and the nucleotides may be any one of A, G, C and U, which are canonical RNAs, and the number of nucleotides combined in each of the two directions may be 4 to 50. Through this process, countless combinations of modified RNA blocks may be produced centered on RNA modification.

In step (b), a modified RNA oligomer may be produced such that 1 to 9 modified RNA blocks are randomly arranged among the countless combinations of modified RNA blocks produced in step (a) above, and through such an arrangement, the diversity of the library according to the present invention may be further improved.

In one aspect, the modified RNA oligomer may be an RNA modification library characterized in that 2 to 9 modified RNA blocks, each of which has 4 to 50 random nucleotides randomly linked in 5' and 3' directions with modified RNA as a center, are randomly arranged sequentially.

In the present invention, step (b) may be producing a modified RNA oligomer, further including an anchor sequence
(i) between each modified RNA block constituting a modified RNA oligomer,
(ii) at a 5' end of a modified RNA block located at a 5' end of a modified RNA block constituting a modified RNA oligomer, and/or
(iii) at a 3' end of a modified RNA block located at a 3' end of a modified RNA block constituting a modified RNA oligomer.

In the present invention, the anchor sequence may have a sequence of 4 nucleotides or more, and when there are 2 or less modified RNA blocks in the modified RNA oligomer, the anchor sequence has a length of 40 to 90% of a length of modified RNA block, and when there are 3 to 9 modified RNA blocks in the modified RNA oligomer, the anchor sequence has a length of 20 to 30% of a length of modified RNA block.

As the length of the modified RNA block increases, the accuracy of a motif decreases. In this case, the length of the anchor sequence may be adjusted as described above to maintain a high accuracy of the motif.

For example, if there are 3 or more modified RNA blocks in the modified RNA oligomer and the modified RNA block length is 21, an anchor length of about 6 is appropriate, and if the modified RNA block length is 31, an anchor length of about 9 is appropriate.

However, if the number of modified RNA blocks is 2 or less, the motif extraction accuracy decreases, and thus, in this case, the length of the anchor sequence may be additionally increased to maintain a high motif accuracy.

For example, if the length of the modified RNA block is 21 and the number of modified RNA blocks is 2, the anchor length is appropriately around 12, and if the number of modified RNA blocks is 1, the anchor length is 18. If the length of the modified RNA block is 31 and the number of modified RNA blocks is 2, the anchor length is appropriately around 18, and if the number of modified RNA blocks is 1, the length is appropriately around 27.

It is desirable to select the sequence of anchors by considering the following factors:
(1) The Levenshtein distance between each anchor must be at least 50% of the anchor length.
(2) When all anchors are combined, the ratio of each nucleotide must be equal, and the ratio of nucleotides within each anchor must also be equal. An equal ratio means a ratio in which the length of each anchor is divided into 4 integer ratios closest to 1:1:1:1.
(3) The average alignment error occurring during nanopore sequencing of all 5-mers constituting each anchor must be less than or equal to the 10^{th} percentile.
(4) When partially random oligomers are produced by using all anchors and sampled random blocks, the average minimum free energy (MFE) thereof must be greater than or equal to the 90^{th} percentile. This is to minimize the generation of RNA secondary structures.
(5) When a partially random oligomer is formed as in (4), the average value of the minimum dimer free energy (dimer MFE) occurring in all possible pairs must be greater than or equal to the 90^{th} percentile. This is to minimize RNA dimer formation.
(6) When all subsequences having 50% of the anchor length are extracted from all anchors, there should be no pairs that are reverse complementary among all possible pairs created from them. This is to minimize RNA duplex formation.

Therefore, the anchor may preferably satisfy one or more of the following conditions:
(1) The Levenshtein distance between each anchor should be at least 50% of the anchor length;
(2) The ratio of each nucleotide (A, G, C, U) within each anchor should not differ by more than 10% from 1:1:1:1;
(3) The average alignment error occurring during nanopore sequencing of all 5-mers constituting each anchor should be less than or equal to the 10^{th} percentile.
(4) When partially random oligomers are produced by using all anchors and sampled random blocks, the average value of their minimum free energy (MFE) should be at least 90^{th} percentile.
(5) When partially random oligomers are generated by using all anchors and sampled random blocks, the average value of the dimer minimum free energy (dimer MFE) occurring in all possible pairs should be greater than or equal to the 90^{th} percentile, and
(6) When all subsequences having 50% of the anchor length are extracted from all anchors, there should be no pairs that are reverse complementary among all possible pairs created from the same.

For example, a set of 4 anchor sequences of 6 nucleotides in length that satisfy the above conditions include {CGACAU, CAGUUA, GUCCAG, GUAGUC}, {CGACAU, AGUCCG, CAGUUA, GUAGUC}, {CGACAU, GUAUCC, UUGACG, AGAGUC} and the like.

In the present invention, a plurality of anchors within the modified RNA oligomer may have the same length or different lengths, and may have the same sequence or different sequences.

In the present invention, step (c) may be producing a modified RNA oligomer concatemer (ligate) by ligating a modified RNA oligomer with an RNA ligase, and the RNA ligase may be used without limitation in type.

In this case, in step (b), a modified RNA oligomer is produced such that 1 to 9 random modified RNA blocks are randomly arranged among one or more modified RNA blocks produced in step (a), and 2 to 4 specified nucleotide combinations may be additionally introduced to a 5' end and the 3' end of the modified RNA oligomer produced in this manner, respectively.

For example, in step (b), a modified RNA oligomer is produced such that 2 to 9 random modified RNA blocks are arranged randomly among the plurality of modified RNA blocks produced in step (a), and 2 to 4 specified nucleotide combinations may be additionally introduced to a 5' end and 3' end of the modified RNA oligomer produced in this manner, respectively.

When such a specified nucleotide combination is introduced, the ligation efficiency may be increased in the subsequent steps of step (c) ligating the modified RNA oligomer to produce a modified RNA oligomer concatemer.

In one aspect, the RNA ligase may be T4 RNA ligase 1, the 2 specified nucleotide combinations may be any one of combinations listed in Table 5 below, the 3 specified nucleotide combinations are any one of combinations listed in Table 6 below, and the 4 specified nucleotide combinations may be any one of the combinations listed in Table 7 below, but the present invention is not limited thereto:

**[Table 5]**

| No | 5' end | 3' end |
|---|---|---|
| 1 | GG | GG |
| 2 | GA | AC |
| 3 | GA | CC |
| 4 | CG | GA |
| 5 | CG | UC |
| 6 | UC | AG |
| 7 | AC | CG |
| 8 | GU | CA |
| 9 | AU | CG |
| 10 | UC | GA |

**[Table 6]**

| No | 5' end | 3' end |
|---|---|---|
| 1 | GAU | GAC |
| 2 | CGA | CGA |
| 3 | CGA | GUC |
| 4 | AUC | ACG |
| 5 | GAC | UCC |
| 6 | CCG | AGU |
| 7 | ACG | CCG |
| 8 | UCC | CAG |
| 9 | GUC | ACA |
| 10 | AGU | UAC |

**[Table 7]**

| No | 5' end | 3' end |
|---|---|---|
| 1 | CGAC | AGUC |
| 2 | GAUC | CGAC |
| 3 | GACG | GUCC |
| 4 | CGAU | CCGA |
| 5 | ACGA | UCCG |
| 6 | CCGA | CAGU |
| 7 | UCCG | ACAG |
| 8 | GUCC | UACA |
| 9 | AGUC | CUAC |
| 10 | CAGU | UCUA |

In another aspect, the RNA ligase may be T4 RNA ligase 2 truncated KQ, the 2 specified nucleotide combinations may be any one of combinations listed in Table 8 below, the 3 specified nucleotide combinations may be any one of combinations listed in Table 9 below, and the 4 specified nucleotide combinations may be any one of combinations listed in Table 10 below, but the present invention is not limited thereto:

**[Table 8]**

| No | 5' end | 3' end |
|---|---|---|
| 1 | AG | CG |
| 2 | GG | UU |
| 3 | GA | CC |
| 4 | GA | UG |
| 5 | AA | GC |
| 6 | GG | CU |
| 7 | GA | CU |
| 8 | GA | CG |
| 9 | GC | UG |
| 10 | GA | GC |

**[Table 9]**

| No | 5' end | 3' end |
|---|---|---|
| 1 | AGA | CCG |
| 2 | AGA | UCG |
| 3 | GCA | UUG |
| 4 | GGC | CUU |
| 5 | GGA | CUU |
| 6 | AGA | ACG |
| 7 | GAG | CCC |
| 8 | GAA | CCC |
| 9 | GCA | CUG |
| 10 | AGA | GCG |

**[Table 10]**

| No | 5' end | 3' end |
|---|---|---|
| 1 | AGAA | CUCG |
| 2 | GGCA | CCUU |
| 3 | AGAA | ACCG |
| 4 | AGAA | UCCG |
| 5 | GGCA | ACUU |
| 6 | AGAA | UUCG |
| 7 | AGAA | GCCG |
| 8 | GGCA | UCUU |
| 9 | AGAA | CACG |
| 10 | AGAA | AUCG |

The present invention enables the production of an RNA modification library in a single ligation reaction.

In the present invention, steps (a) and (b) may be produced by chemical or template-independent enzymatic synthesis known in the present technical field. In this case, step (b) may be produced as a separate process after step (a), or steps (a) and (b) may be produced as a single process.

That is, in the present invention, steps (a) and (b) may be produced in a single process, such as producing a modified RNA oligomer such that 1 to 9 modified RNA blocks are randomly arranged among one or more modified RNA blocks in which 4 to 50 random nucleotides are randomly linked in 5' and 3' directions, respectively, of (a') modified RNA.

In one embodiment of the present invention, steps (a) and (b) were produced by a chemical synthesis method, but it is apparent to those skilled in the art that template-independent enzymatic synthesis is also possible. Template-independent enzymatic synthesis is described in detail in, for example, Wiegand, Daniel J., et al. "Template-independent enzymatic synthesis of RNA oligonucleotides." Nature Biotechnology (2024*)*, and those skilled in the art may implement the present invention by template-independent enzymatic synthesis by referring to the above literature instead of chemical synthesis.

In another aspect, in the present invention, steps (a) and (b) may be produced in a single process, such as producing a modified RNA oligomer such that 2 to 9 modified RNA blocks are randomly arranged among a plurality of modified RNA blocks in which 4 to 50 random nucleotides are randomly linked in 5' and 3' directions of modified RNA, respectively;

In this case, the additional process in step (b) may be applied as is to step (a') as long as they are not mutually contradictory.

In the present invention, step (c) may be ligating at a temperature of about 29°C to about 37°C, for example, about 31°C to about 34°C, and preferably about 32°C to about 33°C, for about 4 hours or more, for example, about 8 hours to about 40 hours, and preferably about 10 hours to about 24 hours, to improve ligation efficiency.

In the present invention, step (c) may also be ligating in a reaction solution including about 11 to about 23% (v/v) of PEG8000, for example, about 13 to about 21% (v/v), and preferably about 15 to 19% (v/v), to improve ligation efficiency.

In the present invention, step (c) may also be ligating in a reaction solution including DMSO to improve ligation efficiency. In this case, the ligating may be performed in a reaction solution including about 20% (v/v) or less of DMSO, for example, about 5 to about 15% (v/v), and preferably about 8 to about 12% (v/v).

Thereafter, in step (d), a modified RNA oligomer concatemer having a length of 2 to 20 ligated units that are suitable for RNA sequencing may be selected.

The step of selecting a modified RNA oligomer concatemer with a specific length is known in the art, and in one aspect, it may be performed by subjecting the modified RNA oligomer concatemer to electrophoresis on a PAGE gel and extracting a modified RNA oligomer concatemer of a desired size, but is not limited to.

Thereafter, in step (e), an RNA tail may be attached to the 3' end of the selected specific length modified RNA oligomer concatemer.

In this case, the RNA tail is attached to improve RNA sequencing efficiency, and means any sequence that has already been clearly specified such that the producer of the RNA modification library can know the exact sequence, and the RNA tail may be a homo or heteropolynucleotide in which about 5 to 150 arbitrary ribonucleotides (A, G, C, U, I) are linked.

The RNA tail may be a homo- or heteropolynucleotide consisting of ribonucleotides selected from any ribonucleotides (A, G, C, U, I), for example, 25 to 100 homo- or heteropolynucleotides, preferably about 25 to 50 homo- or heteropolynucleotides, and more preferably about 25 to 40 homopolynucleotides or heteropolynucleotides.

In one aspect, the RNA tail may be a poly(A) tail, which can be a poly(A) tail consisting of 5 to 150 adenines, for example, 25 to 100 adenines, preferably about 25 to 50 adenines, and more preferably about 25 to 40 adenines.

In another aspect, the RNA tail may be a poly(U) tail, which can be a poly(U) tail consisting of 5 to 150 uracils, for example, 25 to 100 uracils, preferably about 25 to 50 uracils, and more preferably about 25 to 40 uracils.

In still another aspect, the RNA tail may be a poly(I) tail, which can be a poly(I) tail consisting of 5 to 150 inosines, for example, 25 to 100 inosines, preferably about 25 to 50 inosines, and more preferably about 25 to 40 inosines.

In the present invention, the RNA tail may be introduced as a poly(A) tail, a poly(U) tail or a poly(I) tail for the convenience of production. However, if the producer clearly specifies the sequence and can easily produce an adapter or primer based thereon and utilize the same for sequencing, it is not particularly limited in its type, and any ribonucleotide selected from any ribonucleotide (A, G, C, U, I) may be linked to produce a length in the range of about 5 to 150.

In the present invention, in order to poly(A) tail about 25 to 40 adenines, the poly(A) tailing reaction solution may be characterized by including about 5 to 50 uM, for example, about 5 to 25 uM, and preferably about 5 to 15 uM, of ATP.

In this case, the poly(A) tailing reaction may be characterized by reacting for about 30 minutes to about 1 hour, and for example, about 40 minutes to about 50 minutes, by using about 2.5 U to 4 U of E-PAP, for example, and about 3 to 3.5 U of E-PAP.

In one embodiment of the present invention, poly(A) tailing using E-PAP enzyme was performed, but it is apparent to those skilled in the art that poly(U) tailing or poly(I) tailing using poly(U) polymerase may also be performed. Poly(U) tailing or poly(I) tailing is described in detail in, for example, RNA 27 (2021): 1497-1511 and Molecular and Cellular Biology 27.10 (2007): 3612-3624.

Meanwhile, when the RNA tail according to the present invention is a homopolynucleotide or heteropolynucleotide other than a poly(A) tail, a poly(U) tail or a poly(I) tail, the RNA tail may be produced to have a phosphate group at the 5' and 3' ends of 25 to 100 homo or heteropolynucleotides, preferably about 25 to 50 homo or heteropolynucleotides, and more preferably about 25 to 40 homopolynucleotides or heteropolynucleotides (5'p-RNA-3'p), and after pre-adenylation of 5'-p (generation of 5' App-RNA-3'p), it may be attached to the modified RNA oligomer concatemer by ligation.

Meanwhile, a method for producing a canonical RNA library including a plurality of canonical RNA oligomer concatemers having the same structure as a plurality of modified RNA oligomer concatemers included in the RNA modification library, except that the modified RNA is replaced with a corresponding canonical RNA, will be apparent to a person skilled in the art from the above-described method for producing an RNA modification library.

In the present invention, having the same structure means that the library has a structure corresponding to the RNA modification library by producing the canonical RNA library through the same steps and conditions, except that the modified RNA is replaced with a canonicalRNA.

In another aspect, the present invention relates to an RNA modification library produced by the above method.

In still another aspect, the present invention provides an RNA modification library including a plurality of modified RNA oligomer concatemers in the form of 2 to 20 ligated modified RNA oligomers, and
the modified RNA oligomer relates to an RNA modification library, characterized in that 1 to 9 modified RNA blocks, each of which has 4 to 50 random nucleotides randomly linked in the 5' and 3' directions with modified RNA as a center, are randomly arranged consecutively.

For example, the modified RNA oligomer may be a modified RNA block in which 2 to 9 modified RNA blocks in the form in which 4 to 50 random nucleotides are randomly linked in the 5' and 3' directions, respectively, with modified RNA a center, are randomly arranged consecutively.

In the present invention, the modified RNA may be any one modified RNA selected from the group consisting of various modified RNAs described in [Table 1] to [Table 4], but is not limited thereto.

In the present invention, the modified RNA oligomer may further include an anchor sequence
(i) between each modified RNA block constituting a modified RNA oligomer,
(ii) at a 5' end of a modified RNA block located at a 5' end of a modified RNA block constituting a modified RNA oligomer, and/or
(iii) at a 3' end of a modified RNA block located at a 3' end of a modified RNA block constituting a modified RNA oligomer.

In the present invention, the anchor sequence has a sequence of 4 nucleotides or more, and when there are 2 or less modified RNA blocks in the modified RNA oligomer, the anchor sequence may have a length of 40 to 90% of a length of the modified RNA blocks, and when there are 3 to 9 modified RNA blocks in the modified RNA oligomer, the anchor sequence may have a length of 20 to 30% of a length of the modified RNA blocks.

In the present invention, 2 to 4 specified nucleotide combinations may be additionally introduced to a 5' end and 3' end of the modified RNA oligomer, respectively.

In the present invention, the modified RNA oligomer concatemer may additionally include an RNA tail consisting of 5 to 150 arbitrary ribonucleotides at a 3' end thereof.

Since the RNA tail has been described in detail above, the repeated description will be omitted.

In still another aspect, the present invention relates to an RNA library set for training an RNA modification detection model, including:
the RNA modification library; and
a canonical RNA library including a plurality of canonical RNA oligomer concatemers having the same structure as a plurality of modified RNA oligomer concatemers included in the RNA modification library, except that the modified RNA is replaced with a corresponding canonical RNA.

In still another aspect, the present invention relates to a method for training an RNA modification detection model using an RNA library set, including: the RNA modification library; and
a canonical RNA library including a plurality of canonical RNA oligomer concatemers having the same structure as a plurality of modified RNA oligomer concatemers included in the RNA modification library, except that the modified RNA is replaced with a corresponding canonical RNA.

In one aspect, the RNA modification library and the canonical RNA library corresponding to the RNA modification library may be provided as components of a kit.

In this case, the kit may include the RNA modification library and a canonical RNA library corresponding to the RNA modification library, and may include instructions explaining the principles and experimental methods for generating sequencing data using the library set.

In still another aspect, the present invention relates to a sequencing data set, generated by using an RNA library set for training the RNA modification detection model.

In the present invention, the sequencing may be single-molecule RNA sequencing, but is not limited thereto.

The single-molecule RNA sequencing method may be any method known in the art, and may be referred to Nature Methods 15 (2018): 201-206, but is not limited thereto.

For example, the sequencing data set may be for deep learning and/or machine learning training.

Therefore, in still another aspect, the present invention relates to a method for training deep learning or machine learning using the sequencing data set.

In still another aspect, the present invention relates to the sequencing data set for use in the use of training deep learning or machine learning.

The oligomers in the RNA modification library according to the present invention have the following advantages. (1) It can overcome the disadvantages of RNA produced by IVT (the presence of specific RNA modifications at all base positions) and can construct a library in which RNA modifications exist only at desired positions. (2) With the RNA modification as a center, the sequences on both sides are consisting of random sequences with the desired length in all combinations that are not limited to specific motifs. This enables a specific type of RNA modification and a corresponding canonical base library that includes all flanking sequences. Through this, it is possible to overcome the disadvantages of a library using RNA extracted from cells or RNA synthesized by inserting only a few specific motif sequences. (3) The present invention can be applied to the production of various types of RNA modification libraries with the same strategy by simply replacing a specific base in the middle with a different type of RNA modification and synthesizing the same.

The present invention is different from previously reported modified RNA libraries in that it is an RNA modification library capable of reflecting various types of RNA modifications and all flanking sequences.

That is, the RNA modification library according to the present invention has an advantage in that it is not limited to a specific RNA modification and its well-known flanking motif, but includes various RNA modifications and all possible flanking sequences existing in nature. In addition, it may be designed such that RNA modifications are located only at a desired position, thereby overcoming the limitations of RNA produced by IVT, and the flanking sequences can also be designed as random or specific sequences. Additionally, since only one RNA modification can be positioned in the center, there is also an advantage in that there is no need to inevitably exclude a specific combination of flanking sequences to prevent two or more RNA modifications from being included in a library produced by IVT, as in existing research articles.

That is, since the RNA modification library designed according to the present invention include a wide range of combinations of modified RNA oligomer concatemers, it may be used to generate sequencing data (e.g., single-molecule RNA sequencing data) and then utilize the same as training data for an RNA modification detection model.

In addition, since the RNA modification library according to the present invention can introduce RNA modifications at a desired location, it has an advantage in that it is more similar to the transcriptome of an actual organism than a conventionally known RNA modification library (e.g., produced by IVT) and can learn knowledge that is generalizable to an actual organism (e.g., deep learning or machine learning). That is, since the RNA modification library according to the present invention can produce a data set including all combinatorially possible motifs around the modified RNA, bias according to motifs may be removed during training.

Meanwhile, software trained by utilizing sequencing data using the RNA modification library according to the present invention as training data is capable of detecting new RNA modifications with very high accuracy, thereby overcoming the limitations that software trained by using existing RNA modification library-based data may be limited to specific RNA modifications and have low prediction accuracy for other modifications.

Moreover, software trained by using various types of RNA modification library data designed according to the present invention will not only be able to accurately detect RNA modifications present in all flanking sequences, but will also be able to simultaneously detect multiple RNA modifications at one time.

It is known that RNA modification plays a role in regulating biological processes such as embryogenesis, modification and cell fate determination, and it has been consistently reported that the dysregulation of RNA modification is related to various diseases including cancer. Therefore, software utilizing the RNA modification library according to the present invention may be utilized in molecular diagnosis and medical fields related to various diseases. For example, if RNA modification is detected from RNA obtained from various patient samples, the disease-specific RNA modification position may be identified, and by comparing the same with the RNA binding protein position, it will also be possible to identify a new RNA modification protein. In addition, based on the identified RNA modification protein data, biomarker candidates for diagnosing RNA modification-related diseases may be discovered and disease-specific drug targets may be discovered such that it can be actively utilized in the diagnosis and treatment of RNA modification-related diseases.

In addition, software for detecting or predicting modified RNAs utilizing various data sets derived from the RNA modification library according to the present invention may be utilized to newly identify correlations between various RNA modifications and specific biological phenomena and analyze their mechanisms. For example, if software trained by using the RNA modification library according to the present invention is applied to cells during the maternal-to-zygotic transition process at the embryonic development stage, correlations between the corresponding changes over time and various RNA modifications may be newly identified, and new directions may be provided for understanding the mechanisms.

### Example

Hereinafter, the present invention will be described in more detail through examples. It will be apparent to those skilled in the art that these examples are intended only to illustrate the present invention, and the scope of the present invention is not to be construed as being limited by these examples.

### Example 1. Production of RNA modification library

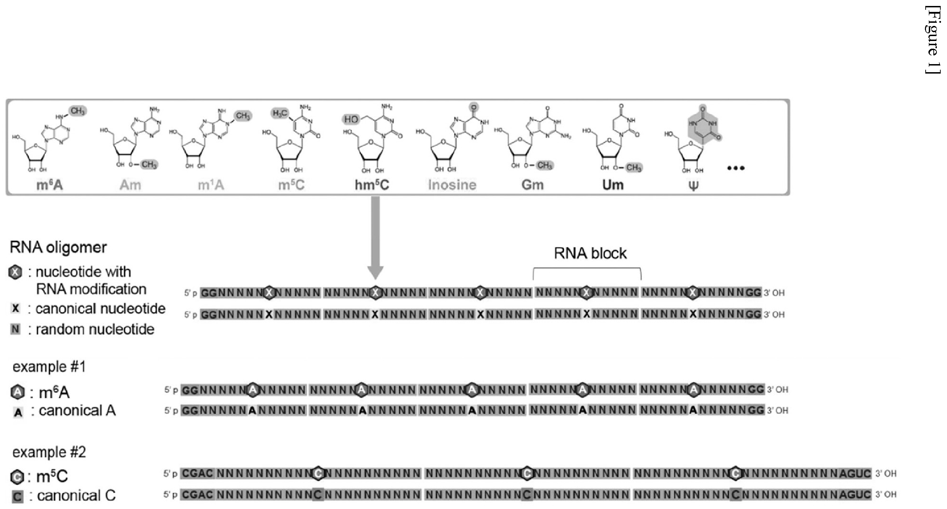

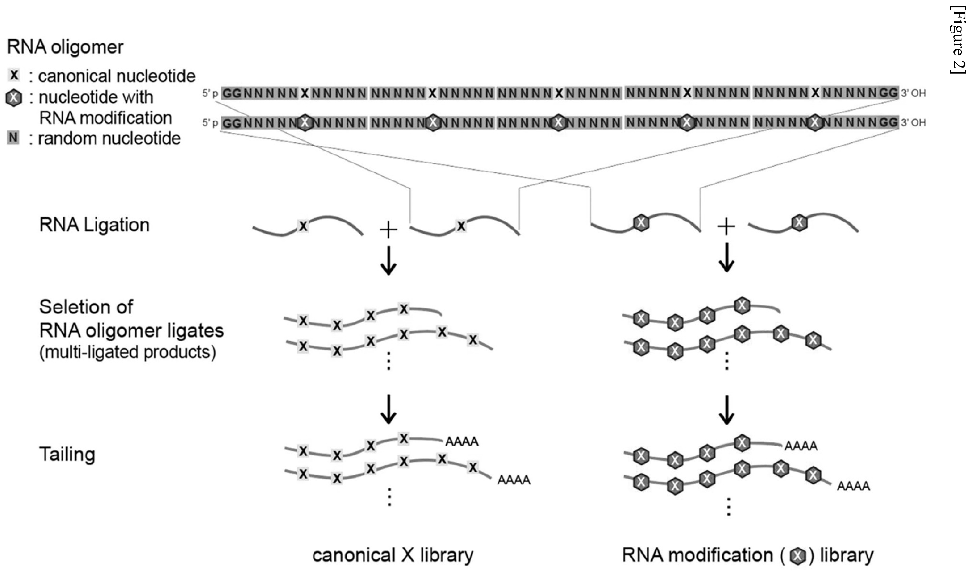

In the present invention, the inventors of the present invention designed a library that considers all flanking sequences for various RNA modifications (FIG. 1), and the specific production process is as follows (FIG. 2).

### (1) Production of modified RNA oligomers with repeated modified RNA blocks and corresponding canonical RNA oligomers

A block was constructed by constructing a sequence consisting of a specific type of RNA modification (or a corresponding common base thereof) and a random (N = A/G/C/U) sequence of 4 to 50 nucleotides on both sides. A modified RNA oligomer including this modified RNA block repeated 1 to 9 times was produced by chemical RNA synthesis (Manufacturer: IDT). However, this modified RNA block may also be produced by a template-independent enzymatic synthesis method.

The 5' and 3' ends of the modified RNA oligomers were designed to have specific combinations of 2 to 4 nucleotides attached to each other. In this case, the optimal nucleotides suitable for the 5' and 3' ends of the modified RNA oligomers were determined through additional experiments.

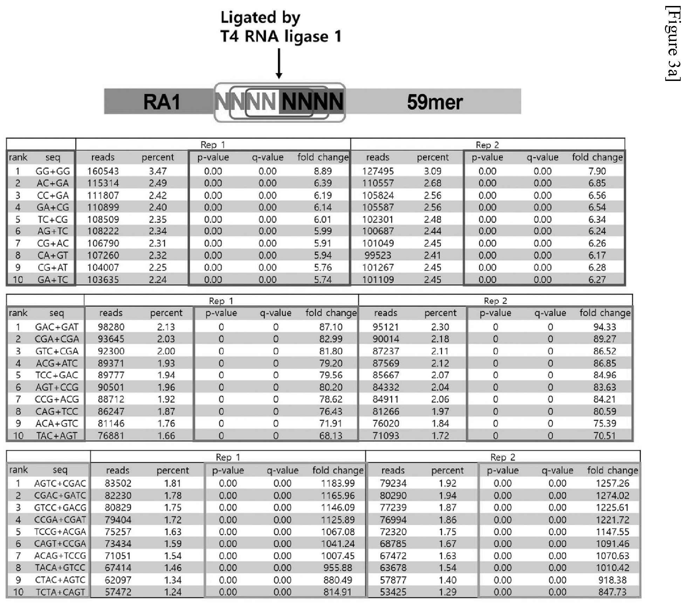

Specifically, an RNA adapter consisting of a 3' end of random nucleotides was designed, and then, RNA ligation was performed at the 5' end of the modified RNA oligomer using T4 RNA ligase 1 (NEB) (FIG. 3a). cDNA was synthesized by using only the ligated RNA, and then, NGS sequencing was performed. The sequence of the ligated position was analyzed, and the combination of the 5' end of the modified RNA oligomer of the most abundant RNA and the 3' end of the adapter sequence was selected. Since these combinations have better reactivity with the T4 RNA ligase 1 enzyme, it was determined that when both ends of the RNA oligomer are designed with the combination, the ligation efficiency and yield in the library production step using the enzyme may be maximized. In addition, this may act as a marker used to find the position of a specific base (X) in the middle after single-molecule sequencing.

The number of reads for the top 10 combinations was measured individually, and the enrichment for each combination (how much more than a random combination) was analyzed to see if it was statistically significant. For two-nucleotide combinations, all of the top 10 combinations showed significant differences (p-value, q-value≒ 0), and it was confirmed that the increase was more than 5.7 times compared to a random combination in both of replicates 1 and 2. For three-nucleotide combinations, all of the top 10 combinations showed significant differences (p-value, q-value≒ 0), and it was confirmed that the increase was more than 68 times compared to a random combination in both of replicates 1 and 2. For the four-nucleotide combinations, all of the top 10 combinations showed significant differences (p-value, q-value ≒ 0), and it was confirmed that the increase was more than 814 times compared to the random combination in both of replicates 1 and 2 (FIG. 3a).

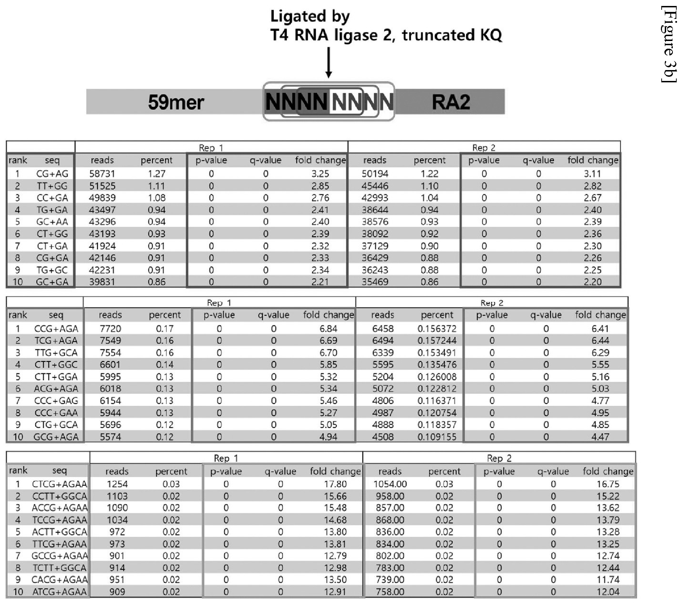

Similarly, an App-RNA adapter consisting of a 5'-end random nucleotide was designed, and ligation was performed to the 3'-end of the modified RNA oligomer by using T4 RNA ligase 2 truncated KQ (NEB) (FIG. 3b). The sequence of the ligated position was analyzed, and the combination of the 3'-end of the modified RNA oligomer of the most abundant RNA and the 5'-end sequence of the adapter was selected. Since these combinations have better reactivity with the T4 RNA ligase 2 truncated KQ enzyme, it was determined that they are combinations that can maximize the ligation efficiency and yield in the library construction step using the enzyme.

The number of reads for the top 10 combinations was measured individually, and the enrichment for each combination (how much more than a random combination) was analyzed to see if it was statistically significant. For two-nucleotide combinations, all of the top 10 combinations showed significant differences (p-value, q-value≒ 0), and it was confirmed that both of replicates 1 and 2 showed a 2.2-fold or more increase compared to a random combination. For three-nucleotide combinations, all of the top 10 combinations showed significant differences (p-value, q-value≒ 0), and it was confirmed that both of replicates 1 and 2 showed a 4.47-fold or more increase compared to a random combination. For the four nucleotide combinations, all of the top 10 combinations showed significant differences (p-value, q-value ≒ 0), and it was confirmed that the increase was more than 12-fold compared to the random combination in both of replicates 1 and 2 (FIG. 3b).

In this way, the inventors of the present invention identified 10 end combinations that maximized ligation efficiency depending on the enzyme to be used for ligation, and inserted 2 to 4 specified nucleotide combinations into the ends of the modified RNA oligomers for subsequent processes.

### (2) RNA oligomer ligation

The above-produced modified RNA oligomers and canonical RNA oligomers were ligated to various lengths by using T4 RNA ligase 1 (NEB), T4 RNA ligase 2 (NEB), or T4 RNA ligase 2 truncated KQ (NEB), respectively, according to the manufacturer's instructions.

### (3) Selection of RNA oligomer concatemers (ligates)

The above-ligated modified RNA oligomers and canonical RNA oligomers were loaded onto an 8% denaturing urea gel, and RNA was separated by length through PAGE (polyacrylamide gel electrophoresis). Among these, bands corresponding to the range of at least 2 to 20 ligated RNA oligomer concatemers were selectively cut out, all were collected, and then eluted to recover RNA from the gel.

### (4) RNA tailing including poly(A) tailing

Poly(A) tailing was performed on the RNA oligomer concatemers having the selected lengths according to the manufacturer's instructions using a poly(A) tailing kit (Invitrogen). Afterwards, the poly(A) tailed RNA oligomer concatemers were recovered by using Dynabeads Oligo(dT) (Invitrogen). Through this process, only linear RNA with poly(A) tails attached was selectively recovered.

In the same manner as above, an RNA modification library including modified RNA concatemers including all of various flanking sequences was produced.

### Example 2. Optimization of process for producing RNA modification library

### 2-1. Selecting the type of ligase

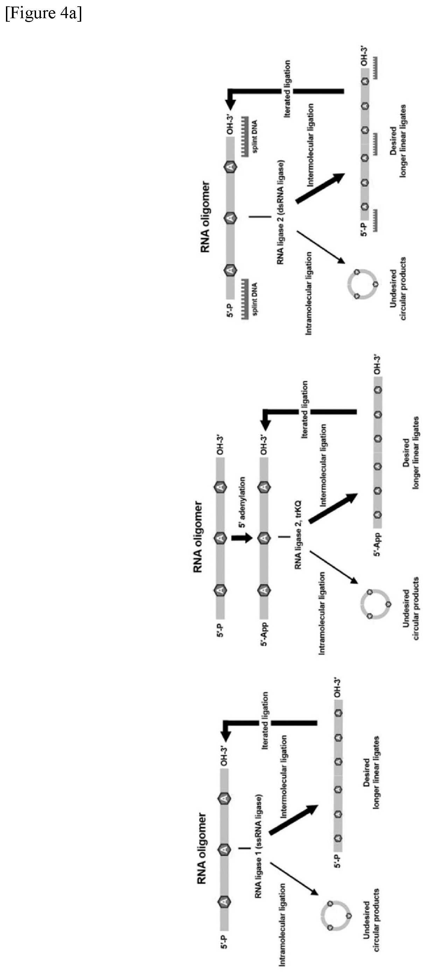

Depending on the type of RNA ligase, the ligation mechanism and the required substrate type are different. Accordingly, ligation was performed by using various RNA ligases, and the most appropriate RNA ligase was selected through confirmation of the results. Tests were performed by using three enzymes: T4 RNA ligase 1 (NEB), T4 RNA ligase 2 (NEB) and T4 RNA ligase 2 truncated KQ (NEB) (FIG. 4a).

T4 RNA ligase 1 is a single-stranded RNA ligase that catalyzes the reaction between the 5' phosphate group and the 3' hydroxyl group of RNA, and thus, the reaction was performed by using modified RNA oligomers without any pretreatment. On the other hand, T4 RNA ligase 2 truncated KQ catalyzes the reaction between the pre-adenylated 5' end and the 3' end of RNA, and thus, the modified RNA oligomers were first 5' adenylated by using Mth RNA ligase (NEB) and then. the reaction was performed. Finally, T4 RNA ligase 2 is a double-stranded RNA ligase that catalyzes the reaction between the 5' phosphate group and the 3' hydroxyl group in double-stranded RNA or RNA-DNA hybrids. Therefore, after synthesizing single-stranded DNA complementary to the terminal sequence of the modified RNA oligomer, the ligation reaction was performed by adding the DNA together. Each reaction was performed according to the manufacturer's instructions.

The following modified RNA oligomers were used in reactions for comparing T4 RNA ligase 1 and T4 RNA ligase 2 truncated KQ.

[NNNN(m⁶A)NNNNNNNN(m⁶A)NNNNNNNN(m⁶A)NNNNNNNN(m⁶A) NNNN] (here, N represents any RNA sequence).

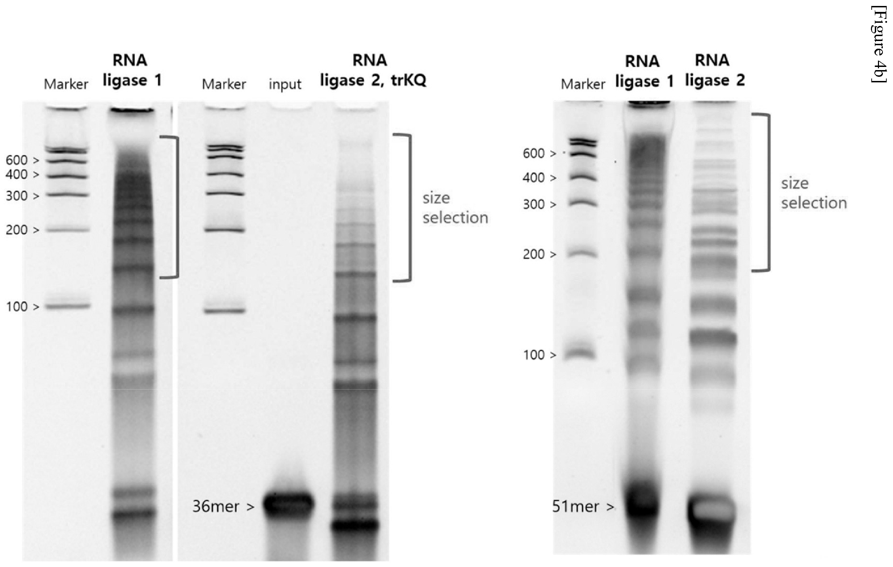

As a result of examining the ligation pattern using the oligomers, it was confirmed through PAGE that relatively more long-length RNA oligomer concatemers were generated when T4 RNA ligase 1 was used. In contrast, in the experiment using T4 RNA ligase 2 truncated KQ, it was confirmed that more short-length concatemers were generated (FIG. 4b).

In addition, the following modified RNA oligomers were used in reactions for comparing T4 RNA ligase 1 and T4 RNA ligase 2.

[CGACAGAUGAGUUCCNNNNNNNNNN(Um)NNNNNNNNNNCCU

UGAUAGACAGUC]

In addition, experiments were conducted by synthesizing the following single-stranded DNA having a sequence complementary to the terminal sequence of the corresponding modified RNA oligomer for RNA ligase 2 reaction.
[TCATCTGTCGGACTGTCTAT]

As a result of examining the ligation pattern using the oligomer, it was confirmed through PAGE that a relatively larger amount of long-length RNA oligomer concatemers were generated when T4 RNA ligase 1 was used. Compared to the RNA ligase 2 experiment, the bands were darker in the long size range, and the amount of remaining input (51mer) was confirmed to be less (FIG. 4b).

### 2-2. Ligation optimization

Through the above experiments, T4 RNA ligase 1 (NEB) was selected among RNA ligases, but when ligation was performed according to the manufacturer's instructions for the enzyme, it was confirmed that the ligation efficiency was still low and the yield was low. Therefore, the inventors of the present invention attempted to establish new ligation conditions suitable for designing an RNA modification library.

Accordingly, the reaction temperature, reaction time, PEG (polyethylene glycol) 8000 concentration and DMSO (dimethyl sulfoxide) concentration were varied, and the amount of poly(A)+ RNA was calculated relative to the amount of input RNA oligomer, and the condition showing the highest yield was selected.

### (Condition 1) Reaction temperature

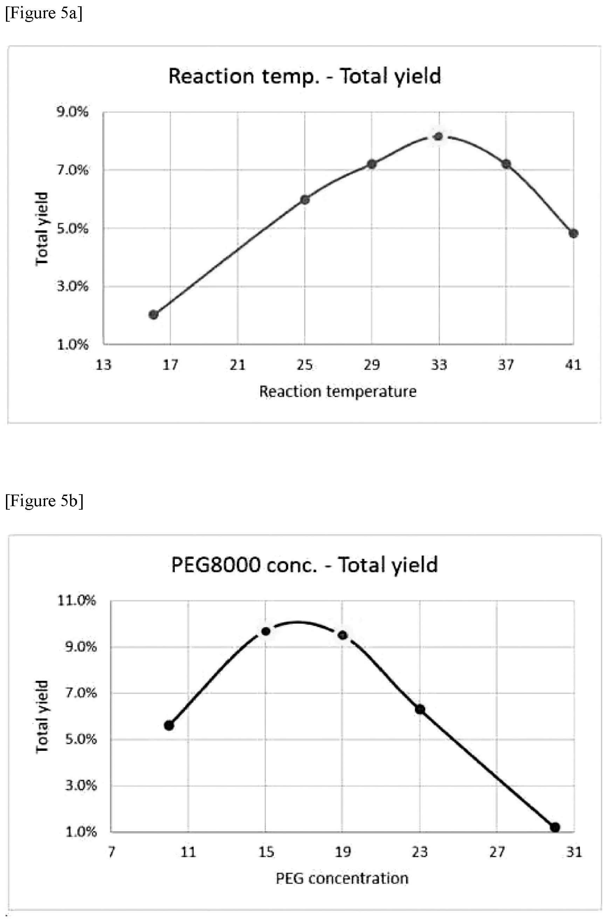

Ligation was performed at temperature conditions of 16, 25, 29, 33, 37 and 41°C for reactants of the same composition. As a result of calculating the yield, it was confirmed that the highest yield was observed at 33°C (FIG. 5a).

### (Condition 2) PEG8000 concentration.

The experiment was performed under conditions where the concentration of PEG8000 in the reaction composition was 10, 15, 19, 23 and 30%. As a result of calculating the yield, it was confirmed that the highest yield was achieved at 15% or 19% (FIG. 5b).

### (Condition 3) DMSO concentration

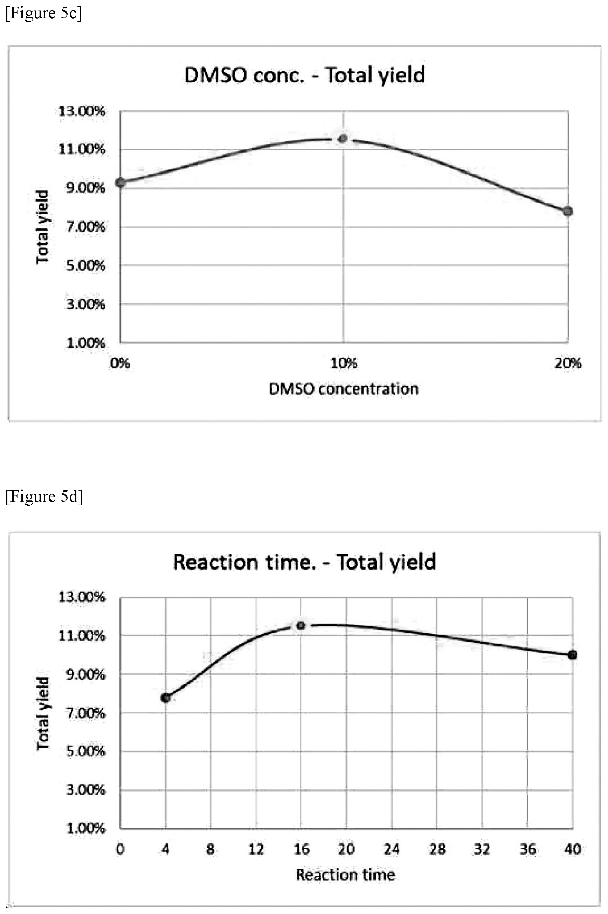

Experiments were performed under conditions of 0, 10 and 20% concentration of DMSO in the reaction composition. As a result of calculating the yield, it was confirmed that the highest yield was observed at 10% DMSO (FIG. 5c).

### (Condition 4) Reaction time

In order to find the most suitable reaction time at the optimal temperature of 33°C, experiments were performed under conditions of 4, 16 and 40 hours. As a result of calculating the yield, it was confirmed that the highest yield was shown in the 16-hour reaction (FIG. 5d).

### (Condition 5) Final condition comparison

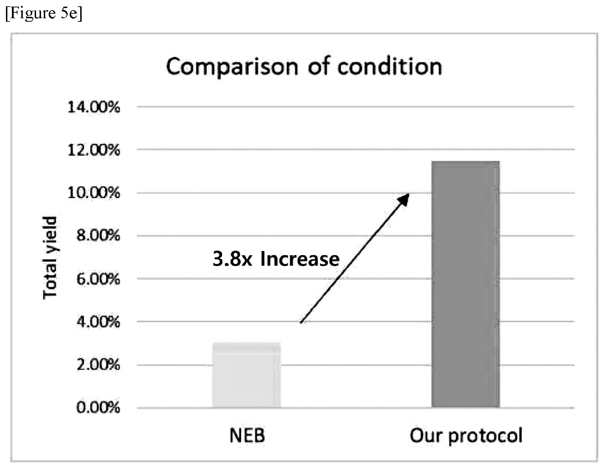

The experiment was performed by using the conditions provided by NEB and the optimized conditions of the present invention. As a result of calculating the yield, it was confirmed that the yield increased 3.8 times from 3% to 11.5% (FIG. 5e).

### 2-3. Optimization of RNA tailing including poly(A) tailing

In order to stabilize the RNA oligomer concatemer and complete the same in the form of a sequence-capable library, an RNA tailing process including poly(A) tailing (addition of non-template nucleotides to the 3' end of RNA) is required. Since the modified RNA oligomer concatemer produced in the present invention is different from mRNA and has more RNAs of relatively short lengths, it was determined that a process of finding a poly(A) tail of a length suitable for the library was necessary first. Therefore, 1) a process of finding the optimal poly(A) tail length was performed, and 2) an optimization process of finding experimental conditions to match the length was performed thereafter.

In the process of finding the appropriate poly(A) tail length, poly(A) tails of 20, 30, 40, 100 and 300 nucleotides in length were generated at the 3' end of the experimental RNAs of the same length with different indices. Afterwards, sequencing was performed by using samples in which each RNA molecule was mixed in the same mole number. In the sequencing results, the poly(A) tail length of the RNA with the highest base quality and mapping degree was selected as the optimal poly(A) tail length.

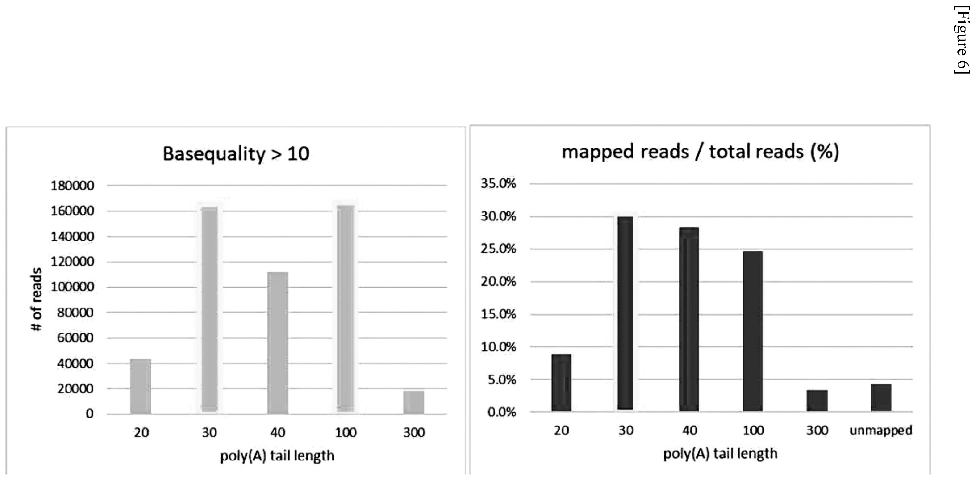

As a result, the base quality showed high results in 30 and 100 nucleotide poly(A) tails, and the ratio of mapped reads was confirmed to be the highest in 30 nucleotide poly(A) tails (FIG. 6). Since the condition that showed the highest value in both results was the poly(A) tail length of 30 nucleotides, the corresponding length was set as the most suitable length for the library of the present invention.

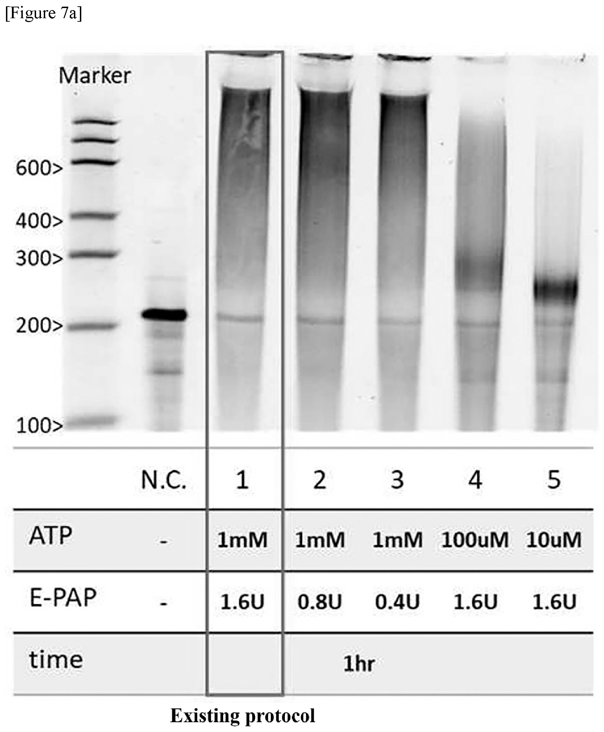

Next, an experimental process was conducted to attach a poly(A) tail of approximately 30 nucleotides using E-PAP (*E. coli* Poly(A) Polymerase) enzyme. When Invitrogen's poly(A) tailing kit was used according to the manufacturer's method, it was confirmed that a very long poly(A) tail was added (FIG. 7a), and conditions for attaching a 30 nucleotide poly(A) tail were established by changing the ATP concentration, E-PAP enzyme amount and reaction time. The results were confirmed on a denaturing gel through PAGE.

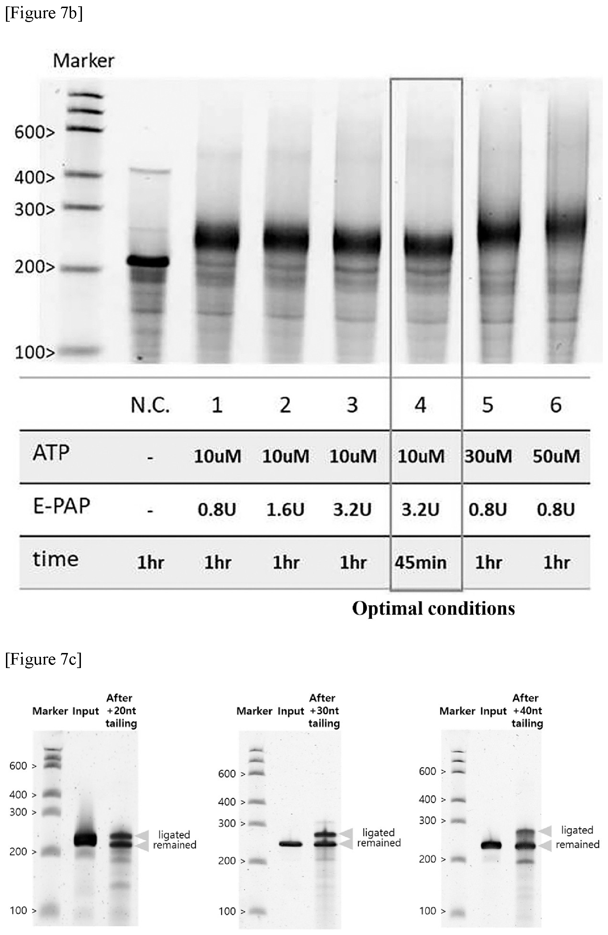

As a result, there was no significant difference even when the amount of E-PAP enzyme was changed when 1 mM ATP was used, and it was confirmed that the length of the poly(A) tail decreased rapidly when ATP was reduced to 1 mM, 100 uM and 10 uM. When the reaction time was reduced to 45 minutes using 3.2 U E-PAP under the 10 uM ATP condition, the result closest to a poly(A) tail of about 30 nucleotides was obtained (FIG. 7b). Through these results, 10 uM ATP, 3.2 U E-PAP and 45 minutes at 37°C were determined to be the optimal conditions for poly(A) tailing for the production of the RNA modification library of the present invention.

This process may be performed by poly(U) tailing or poly(I) tailing using poly(U) polymerase enzyme (NEB). This process may be performed by replacing ATP with UTP or ITP at the same concentration. Afterwards, it is possible to recover the tailed RNA oligomer concatemer using Oligo(dA) or Oligo(dC).

### 2-4. Attachment of RNA tail through ligation

The purpose of performing poly(A) tailing using the E-PAP enzyme is to produce a library consisting of RNA with a form similar to mRNA, because the adapters or RT primers used in the library preparation process generally include oligo(dT). However, if attachment of an RNA tail with an exact length is required, the method through ligation may be preferable, and it may be a more appropriate method for testing various specific experimental conditions (e.g., slight differences in RNA tail length). In addition, the method through ligation is the only method that can be used to attach a tail consisting of a heteropolynucleotide rather than a homopolynucleotide. In this case, the adapter or RT primer with a complementary sequence is designed directly, and the sequencing preparation process is performed.

An RNA oligomer consisting of 20 to 40 nucleotides of As and having phosphate groups attached to both the 5' and 3' ends was designed to be used as an RNA tail. In order to prevent the tails from ligating each other during the ligation reaction to form a circular form of RNA or to prevent multiple tails from being attached to the oligomer concatemer, a phosphate group was also attached to the 3' end. Afterwards, a pre-adenylated App-RNA oligomer was produced by using Mth RNA ligase (NEB), and the reaction was performed. In order to confirm the attachment of the tail of the exact length, RNA tail attachment through ligation was performed at the 3' end of an oligomer having a length of 240 nucleotides.

For the Mth RNA ligase reaction, the reaction was performed at 65°C for 1 hour and 85°C for 5 minutes in a reaction solution including 100 pmol of the enzyme and 100 µM ATP per 100 pmol of RNA oligomer according to the manufacturer's instructions. Afterwards, for the reaction of ligating the oligomer concatemer and the App-RNA oligomer, 40 pmol of App-RNA oligomer, 10% PEG8000 and 200 U of T4 RNA ligase 2 truncated KQ enzyme were added per 20 pmol of RNA substrate according to the manufacturer's instructions, and the reaction was performed at 25°C for 2 hours. As a result of the experiment, it was confirmed that the lengths of the oligomer concatemers with various lengths increased exactly by 20, 30 and 40 nucleotides, and it was confirmed that the degrees of length increase were distinguishable from each other. Therefore, it was determined that the attachment of an RNA tail of an exact length through ligation was successfully achieved (FIG. 7c).

Next, in order to attach a tail that is a heteropolynucleotide rather than a homopolynucleotide, attachment of the RNA tail was performed through ligation.
[GGUACCCGGGCGAAUUCCAAGCUUGAUCGC]

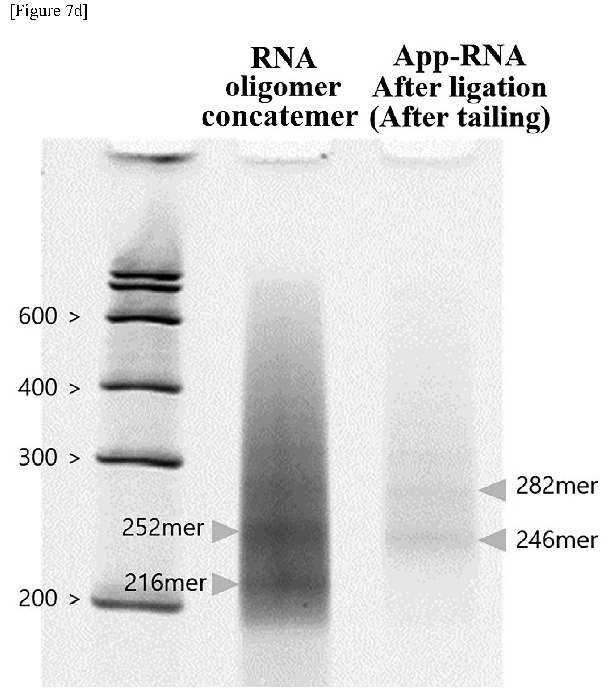

The inventors of the present invention designed an RNA oligomer having the sequence and having phosphate groups attached to both the 5' and 3' ends. Afterwards, pre-adenylated App-RNA oligomers were produced by using Mth RNA ligase (NEB), and the process of performing RNA tailing through ligation to the 3' end of oligomer concatemers of various lengths was performed in the same manner as described above. As a result of the experiment, it was confirmed that the oligomer concatemers with various lengths increased by 30 nucleotides, and therefore, it was determined that the attachment of RNA tail through heteropolynucleotide ligation was successfully performed (FIG. 7d).

The recovery of RNA tails through ligation was accomplished through PAGE, and the bands of the oligomer concatemers with increased length were selectively cut and eluted for recovery. The yield was approximately 10%, which was lower than the yield of approximately 30% for the poly(A) tailing method of Example 2-3. However, it was confirmed through this experiment that it was possible to attach an RNA tail in the form of a heteropolynucleotide. After tailing, the 3' phosphate group was removed through PNK treatment and changed to a 3' hydroxyl group form, and the product was completed in a form that could be used in the subsequent sequencing preparation process.

### Example 3. Sequencing using RNA modification library

As a first example of the present invention, various combinations of RNA blocks were designed in which 12 random nucleotides (A, G, C, U) were randomly linked in both directions centered on m⁶A and its corresponding canonical base A among modified RNA, and various combinations of RNA oligomers were designed such that the various combinations of RNA blocks were randomly arranged twice consecutively. In this experiment, 5'-CGAC and 3'-AGUC were additionally introduced to the 5' end and the 3' end of each RNA oligomer, respectively, to design an RNA oligomer with a total of 58 nucleotides, and the RNA oligomers were produced by a chemical synthesis method.

The canonical A oligomer and m⁶A oligomer were ligated by using T4 RNA ligase 1 (NEB) enzyme, respectively. In this case, the ligation reaction was performed under the following conditions changed from the manufacturer's (NEB) instructions: 16 hours at 33°C in a reaction solution containing 15% PEG8000 and 10% DMSO.

The RNA oligomer concatemers having a length of 232 nucleotides or longer, in which 4 or more of the RNA oligomers were ligated, were selectively recovered from a gel through PAGE. Afterwards, poly(A) tailing was performed by using a poly(A) tailing kit (Invitrogen). In this case, the reaction was performed under the following conditions changed from the manufacturer's (Invitrogen) instructions: 10 uM ATP, 3.2U E-PAP added, reaction at 37°C for 45 minutes.

Afterwards, only linear RNA attached with a poly(A) tail of approximately 30 nucleotides was selectively obtained by using Dynabeads oligo(dT) (Invitrogen).

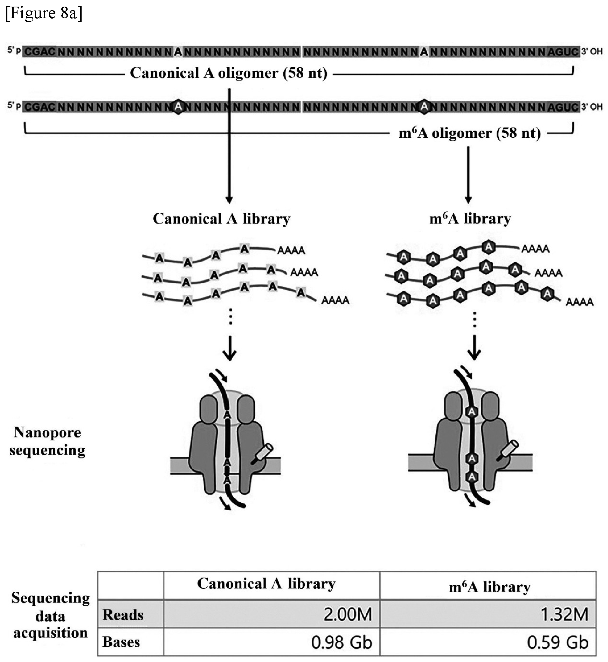

Sequencing was performed by using the produced m⁶A library and the canonical A library. In this case, nanopore sequencing was selected among single-molecule RNA sequencing. Sequencing was performed according to the manufacturer's (Oxford Nanopore) instructions. In the case of the m⁶A library, 1.32 M (million) reads were obtained from one sequencing, and a total of 0.59 Gb of sequencing data was obtained. In the case of the canonical A library, 2.00 M reads were obtained, and a total of 0.98 Gb of sequencing data was obtained (FIG. 8a).

As a second example of the present invention, various combinations of RNA blocks were designed in which 5 random nucleotides (A, G, C, U) were randomly linked in both directions centered on m⁵C and its corresponding canonical base C among the modified RNAs, and various combinations of RNA oligomers were designed such that the various combinations of RNA blocks are randomly arranged five times in succession. An RNA oligomer with a total of 59 nucleotides was produced by a chemical synthesis method by additionally introducing 5'-GG and 3'-GG to the 5' and 3' ends of each RNA oligomer, respectively. An experiment was performed under the same conditions described above using the RNA oligomers, and through this, an m⁵C library and a canonical C library were produced.

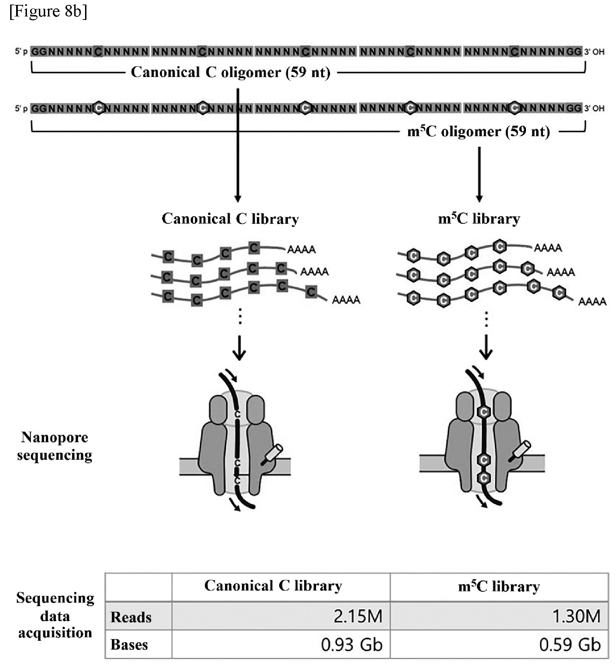

Sequencing was performed by using the two libraries, and nanopore sequencing was selected and performed according to the manufacturer's instructions. For the m⁵C library, 1.30 M (million) reads were obtained from one sequencing run, and a total of 0.59 Gb of sequencing data was obtained. For the canonical C library, 2.15 M reads were obtained, and a total of 0.93 Gb of sequencing data was obtained (FIG. 8b).

### Example 4. Utilization of sequencing data set as training data

The RNA modification library designed according to the present invention has the characteristic of including various modified RNAs and a wide range of combinations of the flanking sequences thereof. Therefore, the sequencing data of this library (e.g., single-molecule RNA sequencing data) was intended to be utilized as training data for an RNA modification detection model.

Motifs (modified RNA or canonical base and flanking sequences thereof; RNA blocks) were extracted from the sequencing reads obtained from each modified RNA and canonical base library. This process was performed by using a motif extraction algorithm using DAG (Directed Acyclic Graph). The algorithm consists of the following steps.

### (1) K-mer indexing and matching

First of all, k-mers (sequences of length k, where k is the length of a specific nucleotide at the end of an oligomer) are indexed from the sequenced reads in the read. In this case, modification, deletion and insertion of the sequence are allowed, but penalties are imposed for each case, and the combined penalty for each k-mer is called the specific-sequence penalty. Afterwards, the indexed k-mers are matched for each specific sequence.

### (2) DAG constitution

A DAG is constructed in which nodes are specific sequences and edges represent valid transitions between specific sequences. Whether each transition is valid is determined by whether the actual transition interval matches the closest transition interval among several transition intervals expected from the specific sequence and the length of the motif within a certain error range. In this case, the penalty calculated from the difference between the actual transition interval and the expected transition interval is called the transition penalty.

### (3) Edge weight application

A weight is calculated and applied to each edge of the DAG created in (2). The weight is calculated by adding the specific-sequence penalty calculated in (1), the transition penalty calculated in (2), and the center-base penalty. The center-base penalty is the minimum number of deletions and insertions required to find a designated center base (one of A, C, G, and U) in the center of the motif.

### (4) Finding the longest path

The longest path is found in the weighted DAG created in (3). In this case, the length of the path is defined as the sum of the weights of each edge. The process of finding the longest path is performed through topology sorting and dynamic programming. After finding the longest path, motifs are extracted from the reads along the path and stored.

By utilizing the above algorithm, motifs were extracted from the sequencing reads of the canonical A and m⁶A libraries of Example 3, and then, the inclusion of motifs was confirmed. It was confirmed that there was a sufficient number of sequencing depths for combinations of 5-mer motifs (256 cases), 7-mer motifs (1,024 cases), and 9-mer motifs (65,536 cases). It was confirmed that both sequencing data showed a depth of at least 10x for all motifs.

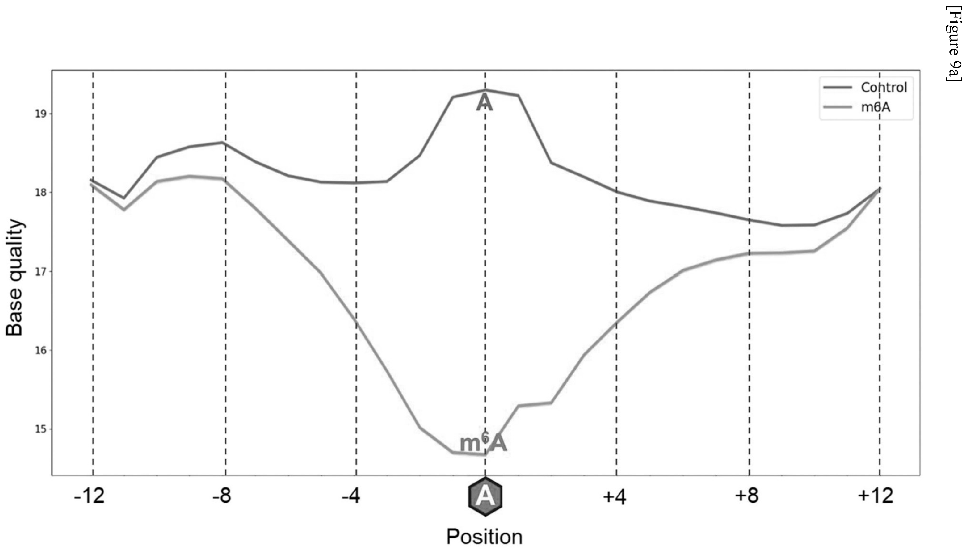

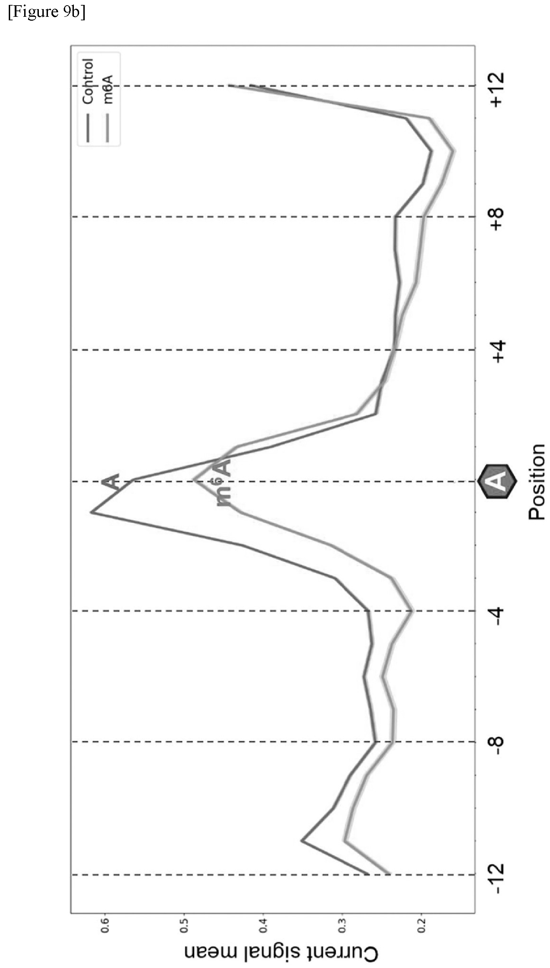

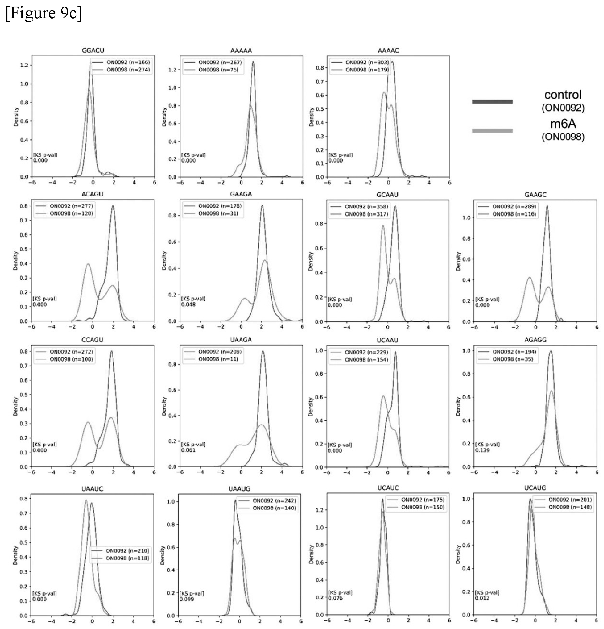

In order to utilize the above data including a wide range of motifs as training data for an RNA modification detection model, the inventors of the present invention attempted to confirm the influence according to the location of the modified RNA on the motif. Compared to canonical RNA, it was confirmed that the base quality of the modified RNA was lower, and particularly, it was confirmed that it greatly decreased around the modified RNA and gradually decreased in the flanking sequences (FIG. 9a). In addition, it was confirmed that the current signals in canonical RNA, modified RNA and the flanking sequences thereof also showed different patterns (FIG. 9b). In particular, it was confirmed that the pattern of the current signal showed different aspects depending on the flanking motif, and it was confirmed that each type of motif showed a specific pattern (FIG. 9c).

Through this analysis, it was confirmed that the library and sequencing data produced in the present invention may be utilized as training data because they include data having various motifs (flanking sequences) and motif-specific patterns.

### Example 5. Improvement of RNA oligomer design through anchor sequence insertion

In order to improve the design of modified RNA oligomers, the inventors of the present invention conducted experiments to increase the accuracy of motifs (modified RNA or canonical bases and the flanking sequences thereof) that can be obtained from sequencing data of each RNA modification and canonical base library.

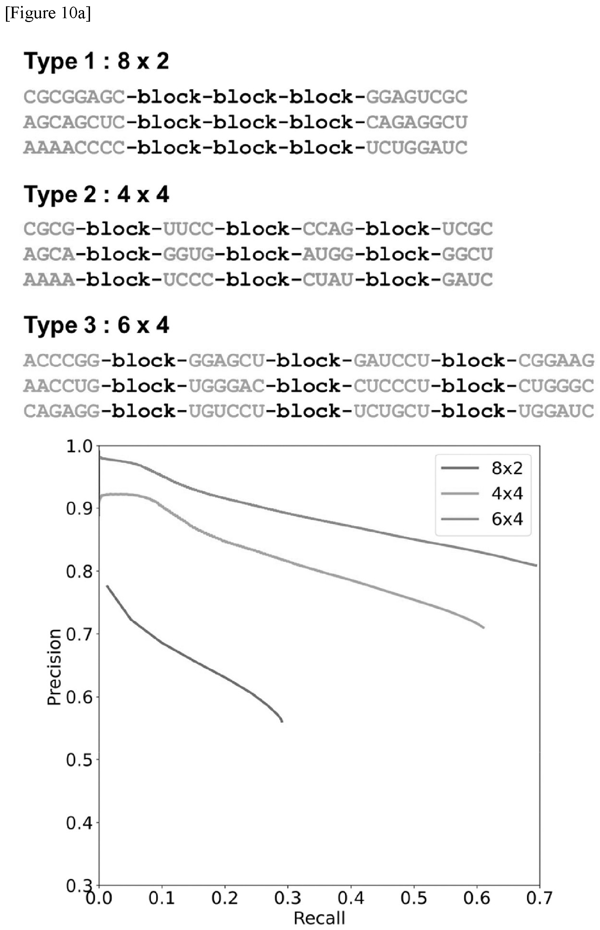

First of all, in order to evaluate the degree of improvement in the design of modified RNA oligomers when inserting an anchor sequence, the motif extraction ability was compared through simulations assuming the case of RNA oligomers with different anchor sequence configurations (FIG. 10a).

As an example, by using the sequencing data of the previous sample, the inventors of the present invention performed a test for the case where a specific anchor sequence of 4 to 6 nucleotides was inserted between blocks.

The data used in this test is the sequencing data of IVT RNA with a length of 274 nucleotides, and the sequence is as follows.

GGGCGAGCUCGGUACCCGGGCGAAUUCCAAGCUUGAUCGCGGAGCUCUAGGA
AGCUCAAACUUUCCUGAUCCUGCGGGCAAGCUAAACCAGUUCCGGAAGAACCUGCGGG
AGUCGCAGCAGCUCCGAUGGGACAGUGGCUGGGUGCAGACUGUGCUCCCUUUGGUUA
UGGACACAUAACUCCUGGGCCAGAGGCUAAAACCCCAGGACCCCUGCUGUCCUUCCCG
CAGCUUCUUCUUGGAGUCUGCUAUAGGCUAGCGACCGUCGAUCUGGAUC

The data are sequence data generated to simulate the case of a modified RNA oligomer concatemer in which three modified RNA oligomers are present (i.e., the modified RNA oligomers are ligated three times) in which a 21 nucleotide-long modified RNA block are repeated three times and included. The motif extraction ability was simulated by using only a specific sequence portion of the data. The specific sequence portion used for extraction under each condition is indicated in bold below.

(Condition 1) When there is no anchor sequence between the modified RNA blocks (a specific sequence of 4 nucleotides was added to the 5' and 3' ends to match the same number of nucleotides as (Condition 2))

GGGCGAGCUCGGUACCCGGGCGAAUUCCAAGCUUGAU
CGCGGAGCUCUAGGAAGCUCAAACUUUCCUGAUCCUGCGGGCAAGCUAAACCAGUUC
CGGAAGAACCUGCGGGAGUCGC/AGCAGCUCCGAUGGGACAGUGGCUGGGUGCAGAC
UGUGCUCCCUUUGGUUAUGGACACAUAACUCCUGGGCCAGAGGCU/AAAACCCCAGG
ACCCCUGCUGUCCUUCCCGCAGCUUCUUCUUGGAGUCUGCUAUAGGCUAGCGACCGU
CGAUCUGGAUC

(Condition 2) When there is an anchor sequence of 4 nucleotides between the modified RNA blocks and the modified RNA blocks.

GGGCGAGCUCGGUACCCGGGCGAAUUCCAAGCUUGAU
CGCGGAGCUCUAGGAAGCUCAAACUUUCCUGAUCCUGCGGGCAAGCUAAACCAGUUC
CGGAAGAACCUGCGGGAGUCGC/AGCAGCUCCGAUGGGACAGUGGCUGGGUGCAGAC
UGUGCUCCCUUUGGUUAUGGACACAUAACUCCUGGGCCAGAGGCU/AAAACCCCAGG
ACCCCUGCUGUCCUUCCCGCAGCUUCUUCUUGGAGUCUGCUAUAGGCUAGCGACCGU
CGAUCUGGAUC

(Condition 3) When there is an anchor sequence of 6 nucleotides between the modified RNA blocks and the modified RNA blocks.

GGGCGAGCUCGGUACCCGGGCGAAUUCCAAGCUUGAU

CGCGGAGCUCUAGGAAGCUCAAACUUUCCUGAUCCUGCGGGCAAGCUAAACCAGUUC

CGGAAG/AACCUGCGGGAGUCGCAGCAGCUCCGAUGGGACAGUGGCUGGGUGCAGAC

UGUGCUCCCUUUGGUUAUGGACACAUAACUCCUGGGC/CAGAGGCUAAAACCCCAGG

ACCCCUGCUGUCCUUCCCGCAGCUUCUUCUUGGAGUCUGCUAUAGGCUAGCGACCGU

CGAUCUGGAUC

In order to calculate the motif selection accuracy for a total of 3 conditions, a precision-recall curve (PR curve) was derived by using the following method. The sequencing data of the IVT RNA above was aligned to the IVT original reference sequence, and the motif extraction algorithm was run on the IVT sequencing data. The motif extraction results were compared with the alignment results, and it was confirmed whether the motif extraction results and the anchor coordinates in the alignment results matched for each anchor position. Next, a precision-recall curve was derived by taking the extraction score of each anchor derived from the motif extraction algorithm as a predicted value and whether the alignment of the anchor coordinates matched as a true value.

From the precision-recall curve, it was confirmed that the motif extraction performance was superior in the order of Condition 3 > Condition 2 > Condition 1. For example, at the same recall of 0.2, Condition 1 showed a precision of less than 0.65, but Condition 3 showed a precision of more than 0.90. From the results, it was confirmed that the greatest improvement was achieved when an anchor sequence of 6 nucleotides was inserted in finding a specific base and motif in the middle of a block in sequencing data.

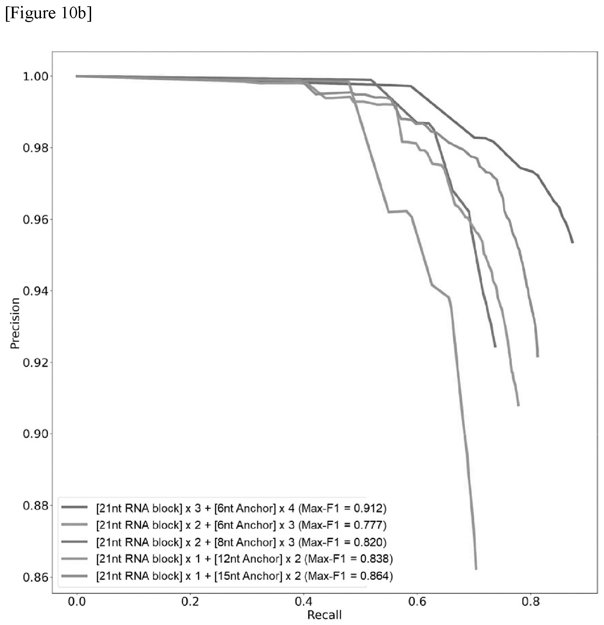

Next, the inventors of the present invention evaluated whether it is necessary to adjust the length of an anchor sequence according to the number of modified RNA blocks. Since the accuracy of motif extraction may decrease when the number of modified RNA blocks decreases to 2 or less, the inventors of the present invention intended to check whether the accuracy of motifs could be maintained high by further increasing the length of the anchor sequence. As previously performed, the motif extraction ability was compared through simulations assuming the case of RNA oligomers in which the number of RNA blocks and the length of the anchor sequence were set differently (FIG. 10b). As in the previous experiment, the inventors of the present invention assumed a case including a modified RNA block with a length of 21 nucleotides, and confirmed the motif extraction performance according to the condition of decreasing the number of RNA blocks.
(Condition 1) When there are three modified RNA blocks and an anchor sequence of 6 nucleotides
(Condition 2) When there are two modified RNA blocks and an anchor sequence of 6 nucleotides
(Condition 3) When there are two modified RNA blocks and an anchor sequence of 8 nucleotides
(Condition 4) When there is one modified RNA block and an anchor sequence of 12 nucleotides
(Condition 5) When there is one modified RNA block and an anchor sequence of 15 nucleotides

As a result of calculating the precision-recall curve for a total of 5 conditions, it was confirmed that when the number of RNA blocks actually decreased, the motif extraction performance decreased significantly when anchor sequences of the same length were used (Condition 1 > Condition 2), and the performance increased when the anchor sequence length increased (Condition 2 > Condition 3, Condition 4 > Condition 5). In addition, it was confirmed that the motif extraction performance can be maintained to some extent by increasing the length of the anchor sequence as the number of RNA blocks decreased (Condition 3 ≒ Condition 4). Finally, it was confirmed that the motif extraction accuracy can be maintained by increasing the length of the anchor sequence by about three times even when the number of RNA blocks is reduced to 1/3 (Condition 1 ≒ Condition 5).

Therefore, it is desirable that the anchor length be 20 to 30% of the length of the modified RNA block, but when the number of RNA blocks is 2 or less, it was confirmed that the motif extraction accuracy can be maintained by increasing the length of the anchor sequence inversely proportional to the number of RNA blocks.

Next, the sequence constitution of the anchors was selected by considering the following factors.
(1) The Levenshtein distance between each anchor must be 50% or more of the anchor length.
(2) When all anchors are combined, the ratio of each nucleotide must be equal, and the ratio of nucleotides within each anchor must also be equal. An equal ratio means a ratio in which the length of each anchor is divided into 4 integer ratios closest to 1:1:1:1.
(3) The average alignment error occurring during nanopore sequencing of all 5-mers constituting each anchor must be less than or equal to the 10^{th} percentile.
(4) When partially random oligomers are created by using all anchors and sampled random blocks, their average minimum free energy (MFE) must be greater than or equal to the 90^{th} percentile. This is to minimize the creation of RNA secondary structures.
(5) When partially random oligomers are formed as in (4), the average value of the minimum dimer free energy (dimer MFE) occurring in all possible pairs must be greater than the 90^{th} percentile. This is to minimize RNA dimer formation.
(6) When all subsequences having 50% of the anchor length are extracted from all anchors, there should be no pairs that are reverse complementary among all possible pairs created from them. This is to minimize RNA duplex formation.

The following set of examples of anchor sequences with 6 nucleotides in length satisfying the above conditions was derived.

{CGACAU, CAGUUA, GUCCAG, GUAGUC}, {CGACAU, AGUCCG, CAGUUA,
GUAGUC}, {CGACAU, GUAUCC, UUGACG, AGAGUC}, {CGACAU, CCAUUG, AAGCGU,
GUAGUC}

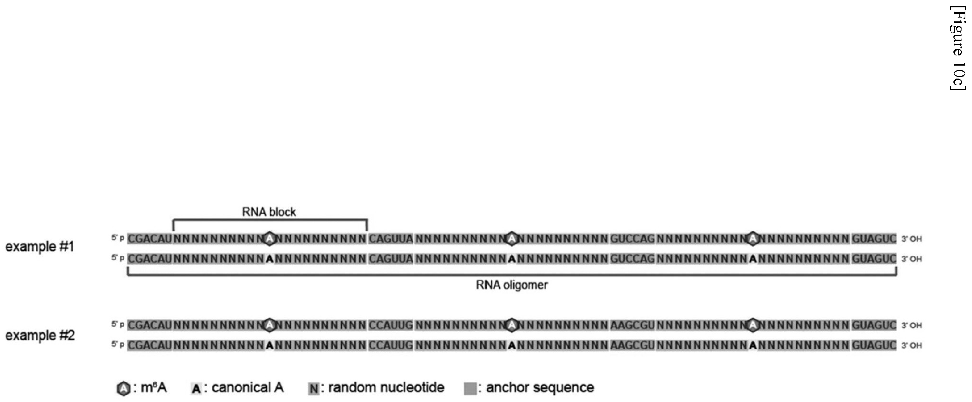

By using the derived anchor sequence, an anchor sequence having a specific sequence was inserted between the modified RNA blocks and blocks in the modified RNA oligomer design of Example 1 (FIG. 10c). As a result of using the design with the anchor sequence inserted, it was confirmed that the motif extraction accuracy increased from 66.5% to 99.7%.

As described above, specific parts of the present invention have been described in detail. It will be apparent to those skilled in the art that such specific descriptions are merely preferred embodiments and the scope of the present invention is not limited thereby. Accordingly, the actual scope of the present invention will be defined by the appended claims and their equivalents.

### [National Research and Development Project That Supported This Invention]

[Project Identification Number] 1711186079
[Project Number] 2020R1A2C3007032
[Name of Ministry] Ministry of Science and ICT
[Name of Project Management (Specialized) Institution] National Research Foundation of Korea
[Title of Research Project] Individual Basic Research (Ministry of Science and ICT) - Mid-Career Research Project
[Title of Research Task] Discovery of New Type of Cancer-Caused Noncoding Mutation through Identification of Regulatory Mechanism of MicroRNA Targeting by RNA-Binding Proteins
[Name of Project Performance Institution] Seoul National University
[Research Period] June 1, 2020 to February 28, 2025

### [National Research and Development Project That Supported This Invention]

[Project Identification Number] 1711200977
[Project Number] 2022M3A9I2082294
[Name of Ministry] Ministry of Science and ICT
[Name of Project Management (Specialized) Institution] National Research Foundation of Korea
[Title of Research Project] Biomedical Technology Development Project
[Title of Research Task] (Joint 3) Establishment of Model to Predict the Occurrence of COVID-19 Virus Variants and
Development of Integrated Mechanism Analysis-based Prevention Platform Technology
[Name of Project Performance Institution] Seoul National University
[Research Period] April 1, 2022 to December 31, 2026

### [National Research and Development Project That Supported This Invention]

[Project Identification Number] 1711200846
[Project Number] 2019M3E5D3073104
[Name of Ministry] Ministry of Science and ICT
[Name of Project Management (Specialized) Institution] National Research Foundation of Korea
[Title of Research Project] Omics-based Precision Medical Technology Development Project - Biomedical Technology Development Project
[Title of Research Task] Development of Exosome Multi-Omics Analysis Platform for Precision Medicine for Diabetic Complications
[Name of Project Performance Institution] Seoul National University
[Research Period] July 1, 2019 to December 31, 2024

### [National Research and Development Project That Supported This Invention]

[Project Identification Number] 1711188895
[Project Number] 2020R1A5A1018081
[Name of Ministry] Ministry of Science and ICT
[Name of Project Management (Specialized) Institution] National Research Foundation of Korea
[Title of Research Project] Group Research Support
[Title of Research Task] Systems Aging Mechanism Research Center
[Name of Project Performance Institution] Seoul National University
[Research Period] July 1, 2020 to February 28, 2027

## Claims

1. A method for producing an RNA modification library, comprising:
(a) preparing one or more modified RNA blocks in which 4 to 50 random nucleotides are randomly linked to each of 5' and 3' directions of a modified RNA;
(b) preparing a modified RNA oligomer in which 1 to 9 modified RNA blocks are randomly arranged among the one or more modified RNA blocks;
(c) ligating the modified RNA oligomers to prepare a modified RNA oligomer concatemer;
(d) selecting a modified RNA oligomer concatemer in which 2 to 20 of the modified RNA oligomers are ligated;
(e) attaching an RNA tail consisting of 5 to 150 arbitrary ribonucleotides to the selected modified RNA oligomer concatemer; and
(f) recovering the modified RNA oligomer concatemer to which the RNA tail is attached.

2. The method of claim 1, wherein the modified RNA is any one modified RNA selected from the group consisting of:
(1) A modification of Table [1] below:
**[Table 1]**
| | **Name** | **Short Name** |
|---|---|---|
| 1 | 2-methylthio-N6-methyladenosine | ms²m⁶A |
| 2 | N6-(cis-hydroxyisopentenyl)adenosine | io⁶A |
| 3 | N6-methyl-N6-threonylcarbamoyladenosine | m6t⁶A |
| 4 | N6-hydroxynorvalylcarbamoyladenosine | hn⁶A |
| 5 | 2'-O-ribosyladenosine (phosphate) | Ar(p) |
| 6 | 1-methylinosine | m¹I |
| 7 | 1,2'-O-dimethyladenosine | m¹Am |
| 8 | 1,2'-O-dimethylinosine | m¹Im |
| 9 | 2-methyladenosine | m²A |
| 10 | N6,N6-dimethyladenosine | m⁶₂A |
| 11 | 2-methylthio-N6-isopentenyladenosine | ms²i⁶A |
| 12 | 2-methylthio-N6-threonylcarbamoyladenosine | ms²t⁶A |
| 13 | N6-isopentenyladenosine | i⁶A |
| 14 | 2-methylthio-N6-hydroxynorvalylcarbamoyladenosine | ms²hn⁶A |
| 15 | N6,2'-O-dimethyladenosine | m⁶Am |
| 16 | 1-methyladenosine | m¹A |
| 17 | N6-methyladenosine | m⁶A |
| 18 | inosine | I |
| 19 | N6-glycinylcarbamoyladenosine | g⁶A |
| 20 | N6,N6,2'-O-trimethyladenosine | m⁶zAm |
| 21 | N6-acetyladenosine | ac⁶A |
| 22 | 2-methylthio-N6-(cis-hydroxyisopentenyl) adenosine | ms²io⁶A |
| 23 | 2'-O-methyladenosine | Am |
| 24 | 2'-O-methylinosine | Im |
| 25 | N6-threonylcarbamoyladenosine | t⁶A |
| 26 | 8-methyladenosine | m⁸A |
| 27 | 2,8-dimethyladenosine | m^{2,8}A |
| 28 | cyclic N6-threonylcarbamoyladenosine | ct⁶A |
| 29 | N6-hydroxymethyladenosine | hm⁶_{A} |
| 30 | N6-formyladenosine | f⁶A |
| 31 | 2-methylthio cyclic N6-threonylcarbamoyladenosine | ms²ct⁶A |
| 32 | hydroxy-N6-threonylcarbamoyladenosine | ht⁶A |
| 33 | 2- methylthiomethylenethio-N6-isopentenyl-adenosine | msms²i⁶A |
(2) C modification of [Table 2] below:
**[Table 2]**
| | **Name** | **Short Name** |
|---|---|---|
| 1 | 5,2'-O-dimethylcytidine | m⁵Cm |
| 2 | N4-methylcytidine | m⁴C |
| 3 | N4,N4-dimethylcytidine | m⁴₂C |
| 4 | 5-methylcytidine | m⁵C |
| 5 | 5-formylcytidine | f⁵C |
| 6 | N4,2'-O-dimethylcytidine | m⁴Cm |
| 7 | 3-methylcytidine | m³C |
| 8 | N4,N4,2'-O-trimethylcytidine | m⁴₂Cm |
| 9 | N4-acetylcytidine | ac⁴C |
| 10 | 2'-O-methylcytidine | Cm |
| 11 | 2-thiocytidine | s²C |
| 12 | 2-lysidine | k²C |
| 13 | 5-formyl-2'-O-methylcytidine | f⁵Cm |
| 14 | N4-acetyl-2'-O-methylcytidine | ac⁴Cm |
| 15 | 5-hydroxymethylcytidine | hm⁵C |
| 16 | agmatidine | C+ |
| 17 | 5-hydroxycytidine | ho⁵C |
| 18 | 2'-O-methyl-5-hydroxymethylcytidine | hm⁵Cm |
(3) G modification of [Table 3] below:
**[Table 3]**
| | **Name** | **Short Name** |
|---|---|---|
| 1 | N2,7-dimethylguanosine | m^{2,7}G |
| 2 | epoxyqueuosine | oQ |
| 3 | N2,N2-dimethylguanosine | m²₂G |
| 4 | 1-methylguanosine | m¹G |
| 5 | 2'-O-methylguanosine | Gm |
| 6 | wybutosine | yW |
| 7 | 2'-O-ribosylguanosine (phosphate) | Gr(p) |
| 8 | N2-methylguanosine | m²G |
| 9 | N2,N2,2'-O-trimethylguanosine | m²₂Gm |
| 10 | glutamyl-queuosine | gluQ |
| 11 | methylwyosine | mimG |
| 12 | 7-aminomethyl-7-carbaguanine | preQ1base |
| 13 | 1,2'-O-dimethylguanosine | m¹Gm |
| 14 | queuosine | Q |
| 15 | 7-aminocarboxypropyl-demethylwyosine | yW-86 |
| 16 | 7-aminocarboxypropylwyosine | yW-72 |
| 17 | galactosyl-queuosine | galQ |
| 18 | isowyosine | imG2 |
| 19 | mannosyl-queuosine | manQ |
| 20 | hydroxywybutosine | OHyW |
| 21 | archaeosine | G+ |
| 22 | N2,2'-O-dimethylguanosine | m²Gm |
| 23 | 4-demethylwyosine | imG-14 |
| 24 | N2,N2,7-trimethylguanosine | m²₂⁷G |
| 25 | 7-aminocarboxypropylwyosine methyl ester | yW-58 |
| 26 | 7-methylguanosine | ₘ⁷G |
| 27 | 7-cyano-7-deazaguanosine | preQ0 |
| 28 | peroxywybutosine | o₂yW |
| 29 | 7-aminomethyl-7-deazaguanosine | preQ1 |
| 30 | wyosine | imG |
| 31 | N2,7,2'-O-trimethylguanosine | m^{2,7}Gm |
and
(4) U modification of [Table 4] below:
**[Table 4]**
| | **Name** | **Short Name** |
|---|---|---|
| 1 | 5-formyluridine | f⁵U |
| 2 | 5-methyl-2-thiouridine | m⁵s²U |
| 3 | 4-thiouridine | s⁴U |
| 4 | 1-methyl-3-(3-amino-3-carboxypropyl)pseudouridine | m¹acp³Y |
| 5 | 5-carboxymethyl-2-thiouridine | cm⁵s²U |
| 6 | 5-methyluridine | m⁵U |
| 7 | dihydrouridine | D |
| 8 | 5-methyldihydrouridine | m⁵D |
| 9 | 5-taurinomethyluridine | tm⁵U |
| 10 | 5-carboxymethylaminomethyl-2'-O-methyluridine | cmnm⁵Um |
| 11 | 5-formyl-2' | f⁵Um |
| 12 | 5-methoxycarbonylmethyluridine | mcm⁵U |
| 13 | 5-carbamoylmethyl-2'-O-methyluridine | ncm⁵Um |
| 14 | 5-aminomethyl-2-thiouridine | nm⁵s²U |
| 15 | 5-carbamoylmethyluridine | ncm⁵U |
| 16 | 5-carboxymethyluridine | cm⁵U |
| 17 | 5-carboxymethylaminomethyl-2-thiouridine | cmnm⁵s²U |
| 18 | uridine 5-oxyacetic acid | cmo⁵U |
| 19 | 3-methylpseudouridine | m³Y |
| 20 | 3-methyluridine | m³U |
| 21 | 2'-O-methyluridine | Um |
| 22 | 3-(3-amino-3-carboxypropyl)methyluridine | acp³Uₘ |
| 23 | 5-carboxymethylaminomethyl-2-selenouridine | cmnm⁵se²U |
| 24 | 3,2'-O-dimethyluridine | m³Um |
| 25 | uridine 5-oxyacetic acid methyl ester | mcmo⁵U |
| 26 | 5-aminomethyl-2-selenouridine | nm⁵se²U |
| 27 | 5-hydroxyuridine | ho⁵U |
| 28 | 5-formyl-2-thiouridine | f⁵s²U |
| 29 | 5-methoxycarbonylmethyl-2-thiouridine | mcm⁵s²U |
| 30 | 5-carboxyhydroxymethyluridine | chm⁵U |
| 31 | 5-methoxyuridine | mo⁵U |
| 32 | 3-(3-amino-3-carboxypropyl)uridine | acp³U |
| 33 | 5-methylaminomethyl-2-selenouridine | mnm⁵se²U |
| 34 | 5-methoxycarbonylmethyl-2'-O-methyluridine | mcm⁵Um |
| 35 | 5-(carboxyhydroxymethyl)uridine methyl ester | mchm⁵U |
| 36 | 5-carboxymethylaminomethyluridine | cmnm⁵U |
| 37 | 1-methylpseudouridine | m¹Y |
| 38 | 5-taurinomethyl-2-thiouridine | tm⁵s²U |
| 39 | 2-selenouridine | se²U |
| 40 | 2-thiouridine | s²U |
| 41 | 2'-O-methylpseudouridine | Ym |
| 42 | 5-formyl-2-selenouridine | f⁵se²U |
| 43 | 5-methylaminomethyl-2-thiouridine | mnm⁵s²U |
| 44 | 5-aminomethyluridine | nm⁵U |
| 45 | 2-thio-2'-O-methyluridine | s²Um |
| 46 | pseudouridine | Y |
| 47 | 5,2'-O-dimethyluridine | m⁵Um |
| 48 | 5-methylaminomethyluridine | mnm⁵U |
| 49 | 3-(3-amino-3-carboxypropyl)-5,6-dihydrouridine | acp³D |
| 50 | 3-(3-amino-3-carboxypropyl)pseudouridine | acp³Y |
| 51 | 5-(isopentenylaminomethyl)uridine | inm³U |
| 52 | 5-(isopentenylaminomethyl)-2'-O-methyluridine | inm⁵Um |
| 53 | 5-(isopentenylaminomethyl)-2-thiouridine | inm⁵s²U |
| 54 | 5-carbamoylmethyl-2-thiouridine | ncm⁵s²U |
| 55 | 5-carbamoylhydroxymethyluridine | nchm⁵U |
| 56 | 5-(carboxyhydroxymethyl)-2'-O-methyluridine methyl ester | mchm⁵Um |
| 57 | 2-geranylthiouridine | ges²U |
| 58 | 5-carboxymethylaminomethyl-2-geranylthiouridine | cmnm⁵ges²U |
| 59 | 5-methylaminomethyl-2-geranylthiouridine | mnm⁵ges²U |
| 60 | 5-aminomethyl-2-geranylthiouridine | nm⁵ges²U |
| 61 | 5-cyanomethyluridine | cnm⁵U |
| 62 | 2'-O-methyluridine 5-oxyacetic acid methyl ester | mcmo⁵Um |

3. The method of claim 1, wherein step (b) is producing a modified RNA oligomer, further comprising an anchor sequence
(i) between each modified RNA block constituting a modified RNA oligomer,
(ii) at a 5' end of a modified RNA block located at a 5' end of a modified RNA block constituting a modified RNA oligomer, and/or
(iii) at a 3' end of a modified RNA block located at a 3' end of a modified RNA block constituting a modified RNA oligomer.

4. The method of claim 3, wherein the anchor sequence has a sequence of 4 nucleotides or more, and when there are 2 or less modified RNA blocks in the modified RNA oligomer, the anchor sequence has a length of 40 to 90% of a length of a modified RNA block, and when there are 3 to 9 modified RNA blocks in the modified RNA oligomer, the anchor sequence has a length of 20 to 30% of a length of a modified RNA block.

5. The method of claim 1, wherein step (c) is ligating with an RNA ligase.

6. The method of claim 1, wherein step (b) is producing by additionally introducing 2 to 4 specified nucleotide combinations to a 5' end and 3' end of the modified RNA oligomer, respectively.

7. The method of claim 6, wherein the RNA ligase is T4 RNA ligase 1, the 2 specified nucleotide combinations are any one of combinations listed in Table 5 below, the 3 specified nucleotide combinations are any one of combinations listed in Table 6 below, and the 4 specified nucleotide combinations are any one of the combinations listed in Table 7 below,
**[Table 5]**
| No | 5' end | 3' end |
|---|---|---|
| 1 | GG | GG |
| 2 | GA | AC |
| 3 | GA | CC |
| 4 | CG | GA |
| 5 | CG | UC |
| 6 | UC | AG |
| 7 | AC | CG |
| 8 | GU | CA |
| 9 | AU | CG |
| 10 | UC | GA |
**[Table 6]**
| No | 5' end | 3' end |
|---|---|---|
| 1 | GAU | GAC |
| 2 | CGA | CGA |
| 3 | CGA | GUC |
| 4 | AUC | ACG |
| 5 | GAC | UCC |
| 6 | CCG | AGU |
| 7 | ACG | CCG |
| 8 | UCC | CAG |
| 9 | GUC | ACA |
| 10 | AGU | UAC |
**[Table 7]**
| No | 5' end | 3' end |
|---|---|---|
| 1 | CGAC | AGUC |
| 2 | GAUC | CGAC |
| 3 | GACG | GUCC |
| 4 | CGAU | CCGA |
| 5 | ACGA | UCCG |
| 6 | CCGA | CAGU |
| 7 | UCCG | ACAG |
| 8 | GUCC | UACA |
| 9 | AGUC | CUAC |
| 10 | CAGU | UCUA |
or wherein the RNA ligase is T4 RNA ligase 2 truncated KQ, the 2 specified nucleotide combinations are any one of combinations listed in Table 8 below, the 3 specified nucleotide combinations are any one of combinations listed in Table 9 below, and the 4 specified nucleotide combinations are any one of combinations listed in Table 10 below:
**[Table 8]**
| No | 5' end | 3' end |
|---|---|---|
| 1 | AG | CG |
| 2 | GG | UU |
| 3 | GA | CC |
| 4 | GA | UG |
| 5 | AA | GC |
| 6 | GG | CU |
| 7 | GA | CU |
| 8 | GA | CG |
| 9 | GC | UG |
| 10 | GA | GC |
**[Table 9]**
| No | 5' end | 3' end |
|---|---|---|
| 1 | AGA | CCG |
| 2 | AGA | UCG |
| 3 | GCA | UUG |
| 4 | GGC | CUU |
| 5 | GGA | CUU |
| 6 | AGA | ACG |
| 7 | GAG | CCC |
| 8 | GAA | CCC |
| 9 | GCA | CUG |
| 10 | AGA | GCG |
**[Table 10]**
| No | 5' end | 3' end |
|---|---|---|
| 1 | AGAA | CUCG |
| 2 | GGCA | CCUU |
| 3 | AGAA | ACCG |
| 4 | AGAA | UCCG |
| 5 | GGCA | ACUU |
| 6 | AGAA | UUCG |
| 7 | AGAA | GCCG |
| 8 | GGCA | UCUU |
| 9 | AGAA | CACG |
| 10 | AGAA | AUCG |

8. The method of claim 1, wherein steps (a) and (b) are produced by chemical or template-independent enzymatic synthesis, and step (b) is produced as a separate process after step (a), or steps (a) and (b) are produced as a single process.

9. The method of claim 1, wherein step (c) is ligating at 29°C to 37°C for 8 to 40 hours.

10. The method of claim 1, wherein step (c) is ligating in a reaction solution comprising 13 to 21% (v/v) of PEG8000.

11. The method of claim 1, wherein the RNA tail of step (e) is a poly(A) tail, poly(U) tail or poly(I) tail consisting of 5 to 40 ribonucleotides.

12. The method of claim 11, wherein the poly(A) tail, poly(U) tail or poly(I) tail of step (e) is reacted in a reaction solution comprising 5 to 50 uM of ATP, UTP or ITP, respectively.

13. An RNA modification library, comprising a plurality of modified RNA oligomer concatemers in the form of 2 to 20 ligated modified RNA oligomers,
wherein the modified RNA oligomer is **characterized in that** 1 to 9 modified RNA blocks in which 4 to 50 random nucleotides are randomly linked in 5' and 3' directions, respectively, are randomly arranged consecutively around modified RNA as a center.

14. The RNA modification library of claim 13, wherein the modified RNA is any one of modified RNA selected from the group consisting of:
(1) A modification of [Table 1] below:
**[Table 1]**
| | **Name** | **Short Name** |
|---|---|---|
| 1 | 2-methylthio-N6-methyladenosine | ms²m⁶A |
| 2 | N6-(cis-hydroxyisopentenyl)adenosine | io⁶A |
| 3 | N6-methyl-N6-threonylcarbamoyladenosine | m6t⁶A |
| 4 | N6-hydroxynorvalylcarbamoyladenosine | hn⁶A |
| 5 | 2'-O-ribosyladenosine (phosphate) | Ar(p) |
| 6 | 1-methylinosine | m¹I |
| 7 | 1,2'-O-dimethyladenosine | m¹Am |
| 8 | 1,2'-O-dimethylinosine | m¹Im |
| 9 | 2-methyladenosine | m²A |
| 10 | N6,N6-dimethyladenosine | m⁶₂A |
| 11 | 2-methylthio-N6-isopentenyladenosine | ms²i⁶A |
| 12 | 2-methylthio-N6-threonylcarbamoyladenosine | ms²t⁶A |
| 13 | N6-isopentenyladenosine | i⁶A |
| 14 | 2-methylthio-N6-hydroxynorvalylcarbamoyladenosine | ms²hn⁶A |
| 15 | N6,2'-O-dimethyladenosine | m⁶Am |
| 16 | 1-methyladenosine | m¹A |
| 17 | N6-methyladenosine | m⁶A |
| 18 | inosine | I |
| 19 | N6-glycinylcarbamoyladenosine | g⁶A |
| 20 | N6,N6,2'-O-trimethyladenosine | m⁶₂Am |
| 21 | N6-acetyladenosine | ac⁶A |
| 22 | 2-methylthio-N6-(cis-hydroxyisopentenyl) adenosine | ms²io⁶A |
| 23 | 2'-O-methyladenosine | Am |
| 24 | 2'-O-methylinosine | Im |
| 25 | N6-threonylcarbamoyladenosine | t⁶A |
| 26 | 8-methyladenosine | m⁸A |
| 27 | 2,8-dimethyladenosine | m^{2,8}A |
| 28 | cyclic N6-threonylcarbamoyladenosine | ct⁶A |
| 29 | N6-hydroxymethyladenosine | hm⁶A |
| 30 | N6-formyladenosine | f⁶A |
| 31 | 2-methylthio cyclic N6-threonylcarbamoyladenosine | ms²ct⁶A |
| 32 | hydroxy-N6-threonylcarbamoyladenosine | ht⁶A |
| 33 | 2- methylthiomethylenethio-N6-isopentenyl-adenosine | msms²i⁶A |
(2) C modification of [Table 2] below:
**[Table 2]**
| | **Name** | **Short Name** |
|---|---|---|
| 1 | 5,2'-O-dimethylcytidine | m⁵Cm |
| 2 | N4-methylcytidine | m⁴C |
| 3 | N4,N4-dimethylcytidine | m⁴₂C |
| 4 | 5-methylcytidine | m⁵C |
| 5 | 5-formylcytidine | f⁵C |
| 6 | N4,2'-O-dimethylcytidine | m⁴Cm |
| 7 | 3-methylcytidine | m³C |
| 8 | N4,N4,2'-O-trimethylcytidine | m⁴₂Cm |
| 9 | N4-acetylcytidine | ac⁴C |
| 10 | 2'-O-methylcytidine | Cm |
| 11 | 2-thiocytidine | s²C |
| 12 | 2-lysidine | k²C |
| 13 | 5-formyl-2'-O-methylcytidine | f⁵C^{m} |
| 14 | N4-acetyl-2'-O-methylcytidine | ac⁴Cm |
| 15 | 5-hydroxymethylcytidine | hm⁵C |
| 16 | agmatidine | C+ |
| 17 | 5-hydroxycytidine | ho⁵C |
| 18 | 2'-O-methyl-5-hydroxymethylcytidine | hm⁵Cm |
(3) G modification of [Table 3] below:
**[Table 3]**
| | **Name** | **Short Name** |
|---|---|---|
| 1 | N2,7-dimethylguanosine | m^{2,7}G |
| 2 | epoxyqueuosine | oQ |
| 3 | N2,N2-dimethylguanosine | m²₂G |
| 4 | 1-methylguanosine | m¹G |
| 5 | 2'-O-methylguanosine | Gm |
| 6 | wybutosine | yW |
| 7 | 2'-O-ribosylguanosine (phosphate) | Gr(p) |
| 8 | N2-methylguanosine | m²G |
| 9 | N2,N2,2'-O-trimethylguanosine | m²₂Gm |
| 10 | glutamyl-queuosine | gluQ |
| 11 | methylwyosine | mimG |
| 12 | 7-aminomethyl-7-carbaguanine | preQ1base |
| 13 | 1,2'-O-dimethylguanosine | m¹Gm |
| 14 | queuosine | Q |
| 15 | 7-aminocarboxypropyl-demethylwyosine | yW-86 |
| 16 | 7-aminocarboxypropylwyosine | yW-72 |
| 17 | galactosyl-queuosine | galQ |
| 18 | isowyosine | imG2 |
| 19 | mannosyl-queuosine | manQ |
| 20 | hydroxywybutosine | OHyW |
| 21 | archaeosine | G+ |
| 22 | N2,2'-O-dimethylguanosine | m²Gm |
| 23 | 4-demethylwyosine | imG-14 |
| 24 | N2,N2,7-trimethylguanosine | m²₂⁷G |
| 25 | 7-aminocarboxypropylwyosine methyl ester | yW-58 |
| 26 | 7-methylguanosine | m⁷G |
| 27 | 7-cyano-7-deazaguanosine | preQ0 |
| 28 | peroxywybutosine | o₂yW |
| 29 | 7-aminomethyl-7-deazaguanosine | preQ1 |
| 30 | wyosine | imG |
| 31 | N2,7,2'-O-trimethylguanosine | m^{2,7}Gm |
and
(4) U modification of [Table 4] below:
**[Table 4]**
| | **Name** | **Short Name** |
|---|---|---|
| 1 | 5-formyluridine | f⁵U |
| 2 | 5-methyl-2-thiouridine | m⁵s²U |
| 3 | 4-thiouridine | s⁴U |
| 4 | 1-methyl-3-(3-amino-3-carboxypropyl)pseudouridine | m¹acp³Y |
| 5 | 5-carboxymethyl-2-thiouridine | cm⁵s²U |
| 6 | 5-methyluridine | m⁵U |
| 7 | dihydrouridine | D |
| 8 | 5-methyldihydrouridine | m⁵D |
| 9 | 5-taurinomethyluridine | tm⁵U |
| 10 | 5-carboxymethylaminomethyl-2'-O-methyluridine | cmnm⁵Um |
| 11 | 5-formyl-2' | f⁵U^{m} |
| 12 | 5-methoxycarbonylmethyluridine | mcm⁵U |
| 13 | 5-carbamoylmethyl-2'-O-methyluridine | ncm⁵Um |
| 14 | 5-aminomethyl-2-thiouridine | nm⁵s²U |
| 15 | 5-carbamoylmethyluridine | ncm⁵U |
| 16 | 5-carboxymethyluridine | cm⁵U |
| 17 | 5-carboxymethylaminomethyl-2-thiouridine | cmnm⁵s²U |
| 18 | uridine 5-oxyacetic acid | cmo⁵U |
| 19 | 3-methylpseudouridine | m³Y |
| 20 | 3-methyluridine | m³U |
| 21 | 2'-O-methyluridine | Um |
| 22 | 3-(3-amino-3-carboxypropyl)methyluridine | acp³Um |
| 23 | 5-carboxymethylaminomethyl-2-selenouridine | cmnm⁵se²U |
| 24 | 3,2'-O-dimethyluridine | m³Um |
| 25 | uridine 5-oxyacetic acid methyl ester | mcmo⁵U |
| 26 | 5-aminomethyl-2-selenouridine | nm⁵se²U |
| 27 | 5-hydroxyuridine | ho⁵U |
| 28 | 5-formyl-2-thiouridine | f⁵s²U |
| 29 | 5-methoxycarbonylmethyl-2-thiouridine | mcm⁵s²U |
| 30 | 5-carboxyhydroxymethyluridine | chm⁵U |
| 31 | 5-methoxyuridine | mo⁵U |
| 32 | 3-(3-amino-3-carboxypropyl)uridine | acp³U |
| 33 | 5-methylaminomethyl-2-selenouridine | mnm⁵se²U |
| 34 | 5-methoxycarbonylmethyl-2'-O-methyluridine | mcm⁵Um |
| 35 | 5-(carboxyhydroxymethyl)uridine methyl ester | mchm⁵U |
| 36 | 5-carboxymethylaminomethyluridine | cmnm⁵U |
| 37 | 1-methylpseudouridine | m¹Y |
| 38 | 5-taurinomethyl-2-thiouridine | tm⁵s²U |
| 39 | 2-selenouridine | se²U |
| 40 | 2-thiouridine | s²U |
| 41 | 2'-O-methylpseudouridine | Ym |
| 42 | 5-formyl-2-selenouridine | f⁵se²U |
| 43 | 5-methylaminomethyl-2-thiouridine | mnm⁵s²U |
| 44 | 5-aminomethyluridine | nm⁵U |
| 45 | 2-thio-2'-O-methyluridine | s²Um |
| 46 | pseudouridine | Y |
| 47 | 5,2'-O-dimethyluridine | m⁵Um |
| 48 | 5-methylaminomethyluridine | mnm⁵U |
| 49 | 3-(3-amino-3-carboxypropyl)-5,6-dihydrouridine | acp³D |
| 50 | 3-(3-amino-3-carboxypropyl)pseudouridine | acp³Y |
| 51 | 5-(isopentenylaminomethyl)uridine | inm³U |
| 52 | 5-(isopentenylaminomethyl)-2'-O-methyluridine | inm⁵Um |
| 53 | 5-(isopentenylaminomethyl)-2-thiouridine | inm⁵s²U |
| 54 | 5-carbamoylmethyl-2-thiouridine | ncm⁵s²U |
| 55 | 5-carbamoylhydroxymethyluridine | nchm⁵U |
| 56 | 5-(carboxyhydroxymethyl)-2'-O-methyluridine methyl ester | mchm⁵Um |
| 57 | 2-geranylthiouridine | ges²U |
| 58 | 5-carboxymethylaminomethyl-2-geranylthiouridine | cmnm⁵ges²U |
| 59 | 5-methylaminomethyl-2-geranylthiouridine | mnm⁵ges²U |
| 60 | 5-aminomethyl-2-geranylthiouridine | nm⁵ges²U |
| 61 | 5-cyanomethyluridine | cnm⁵U |
| 62 | 2'-O-methyluridine 5-oxyacetic acid methyl ester | mcmo⁵Um |

15. The RNA modification library of claim 13, wherein the modified RNA oligomer further comprises an anchor sequence
(i) between each modified RNA block constituting a modified RNA oligomer,
(ii) at a 5' end of a modified RNA block located at a 5' end of a modified RNA block constituting a modified RNA oligomer, and/or
(iii) at a 3' end of a modified RNA block located at a 3' end of a modified RNA block constituting a modified RNA oligomer.

16. The RNA modification library of claim 15, wherein the anchor sequence has a sequence of 4 nucleotides or more, and when there are 2 or less modified RNA blocks in the modified RNA oligomer, the anchor sequence has a length of 40 to 90% of a length of a modified RNA block, and when there are 3 to 9 modified RNA blocks in the modified RNA oligomer, the anchor sequence has a length of 20 to 30% of a length of a modified RNA block.

17. The RNA modification library of claim 13, wherein 2 to 4 specified nucleotide combinations are introduced to a 5' end and 3' end of the modified RNA oligomer, respectively.

18. The RNA modification library of claim 13, wherein the modified RNA oligomer concatemer has an additional RNA tail consisting of 5 to 150 arbitrary ribonucleotides attached to a 3' end.

19. An RNA library set for training an RNA modification detection model, comprising:
the RNA modification library according to any one of claims 13 to 18; and
a canonical RNA library comprising a plurality of canonical RNA oligomer concatemers having the same structure as a plurality of modified RNA oligomer concatemers comprised in the RNA modification library, except that the modified RNA is replaced with a corresponding canonical RNA.

20. A sequencing data set, generated by using the RNA library set for training an RNA modification detection model of claim 19.

21. The sequencing data set of claim 20, wherein the sequencing is single-molecule RNA sequencing.

22. The sequencing data set of claim 20, wherein the sequencing data set is for deep learning and/or machine learning training.
